**Europäisches Patentamt**

**European Patent Office**

**Office européen des brevets**

(19)

(11) Veröffentlichungsnummer: **0 179 344**
**B1**

(12)

# EUROPÄISCHE PATENTSCHRIFT

(45) Veröffentlichungstag der Patentschrift:
28.06.89

(21) Anmeldenummer: 85112659.9

(22) Anmeldetag: 07.10.85

(51) Int. Cl.⁴: **C 07 C 41/03**, C 08 G 18/50,
C 08 G 18/67, C 08 G 18/79,
C 08 G 75/04

(54) **Verfahren zur Herstellung von flüssigen, bromhaltigen Alkoxylierungsprodukten und deren Verwendung zur Herstellung von flammwidrigen Polyurethanen.**

(30) Priorität: 20.10.84 DE 3438526

(43) Veröffentlichungstag der Anmeldung:
30.04.86 Patentblatt 86/18

(45) Bekanntmachung des Hinweises auf die Patenterteilung: 28.06.89 Patentblatt 89/26

(84) Benannte Vertragsstaaten:
AT BE CH DE FR GB IT LI NL

(56) Entgegenhaltungen:
DE—A— 2 241 156

(73) Patentinhaber: BAYER AG
D-5090 Leverkusen 1 Bayerwerk (DE)

(72) Erfinder: Dietrich, Manfred, Dr.
Dresdener Strasse 16
D-5090 Leverkusen 1 (DE)
Erfinder: Schmidt, Manfred, Dr.
Zeisigstrasse 5
D-4047 Dormagen (DE)
Erfinder: Wiedermann, Rolf, Dr.
Wiesenstrasse 18
D-5068 Odenthal (DE)
Erfinder: König, Klaus, Dr.
Heymannstrasse 50
D-5090 Leverkusen 1 (DE)

# EP 0 179 344 B1

## Beschreibung

Die Erfindung betrifft ein Verfahren zur Herstellung von lagerstabilen, flüssigen, bromhaltigen, nur geringe Mengen an 2,3-Dibrombutendiol-1,4 aufweisenden, geruchsarmen Alkoxylierungsprodukten und deren Verwendung zur Herstellung von flammwidrigen Polyurethanen, vorzugsweise PU-Schaumstoffen, einschließlich Polyisocyanurat-Schaumstoffen.

Das Verfahren ist dadurch gekennzeichnet, daß man

A) Butindiol-1,4 mit etwa 2 Mol an Epoxiden (vorzugsweise Ethylenoxid und/oder Propylenoxid) in Gegenwart von Katalysatoren aus der Gruppe

a) höhermolekularer Thioether mit einem Molekulargewicht von vorzugsweise 500 bis 5 000, wie sie insbesondere durch Kondensation von Thiodiglykol entstehen, und/oder
b) Thioethern, wie sie durch Anlagerung von Mercaptanen oder Mercaptoalkoholen an ungesättigte Kohlenwasserstoffe erhalten und evtl. durch Kondensation in höhermolekulare Thioether, vorzugsweise mit Molekulargewichten bis 5 000, überführt werden, und/oder
c) Alkalichloriden, insbesondere Kaliumchlorid,

bei erhöhten Temperaturen umsetzt,

B) das Alkoxylierungsprodukt bromiert,

C) den in einer Nebenreaktion entstehenden Bromwasserstoff durch Rückoxidation mit wäßriger Wasserstoffperoxidlösung in die Bromierungsstufe B) rückführt,

D) mit einer höhermolekularen Epoxyverbindung stabilisiert und

E) das bromierte Produkt entwässert.

Bekanntlich finden Polyurethan- und Polyisocyanuratschaumstoffe zahlreiche und unterschiedliche Anwendungen auf den Gebieten des Bauwesens, der Inneneinrichtung und der Isolation. Für solche Anwendungen ist die Flammwidrigkeit der verwendeten Materialien eine notwendige und unerläßliche Eigenschaft. Infolge sich verschärfender gesetzlicher Bestimmungen bezüglich des Brandverhaltens hat sich in den letzten Jahren notwendigerweise auch die Suche nach solchen Werkstoffen verstärkt, die diese strengen gesetzlichen Normen erfüllen.

Es gibt bereits mehrere Mittel, um PU-Schaumstoffe flammfest auszurüsten. Ein bekanntes Verfahren besteht darin, den Schaumstoffen nicht brennbare Zusatzstoffe wie Antimonoxid oder auch halogenierte und/oder phosphorhaltige Verbindungen wie z. B. Tris-(dibrompropyl)-phosphat oder Tris-(dichlorpropyl)-phosphat, chlorierte Biphenyle und halogenierte Kohlenwasserstoffe zuzusetzen. Solche Zusatzstoffe, die nicht in das Polymergerüst eingebaut werden können, ergeben jedoch keine dauerhafte und gleichmäßig verteilte Flammfestigkeit. Darüber hinaus besitzen sie im allgemeinen eine weichmachende Wirkung auf den Schaumstoff und verschlechtern daher seine mechanischen Eigenschaften, insbesondere seine Druckfestigkeit und seine Dimensionsstabilität.

Ein anderes Mittel zur Herstellung von flammwidrigen PU-Schaumstoffen besteht darin, halogenierte und/oder phosphorhaltige Polyole einzusetzen. Hierbei kann das Halogen entweder aromatisch oder aliphatisch gebunden sein. Die Erfahrung hatte jedoch gezeigt, daß aromatisch gebundenes Halogen, wie es z. B. in der DE-OS 23 50 539 beschrieben ist, möglicherweise aufgrund der hohen Dissoziationsenergie der Halogen-Kohlenstoff-Bindung, keinen guten Flammschutz bewirkt. Außerdem besitzen solche Verbindungen einen relativ hohen Schmelzpunkt, der für eine Anwendung sehr hinderlich ist.

In der FR-PS 1 350 425 ist die Verwendung von halogenierten Polyetherpolyolen beschrieben, welche durch Zugabe von Epichlorhydrin auf mehrwertige, monomere Alkohole, welche wenigstens zwei Hydroxylgruppen enthalten, hergestellt wurden. Die aus der Reaktion von organischen Polyisocyanaten mit solchen halogenierten Polyetherpolyolen erhaltenen, zellförmigen Polyurethane weisen zwar bestimmte zufriedenstellende Flammfesteigenschaften auf, jedoch ist ihre Dimensionsstabilität nur mäßig. Solche Polyetherpolyole sind darüber hinaus bei Lagerung in Anwesenheit von Aminverbindungen, welche häufig bei der Herstellung von Polyurethanschäumen verwendet werden, instabil, da die aliphatischen Halogenverbindungen, im Gegensatz zu den aromatischen, sehr leicht Halogenwasserstoff abspalten.

Da man mit Brom als Halogen bekannterweise einen gegenüber Chlor deutlich verbesserten Flammschutzwirkung erzielen kann, wird in der US-PS 37 64 546 ein bromhaltiger Polyether eingesetzt, der durch Alkoxylierung von Dibrombutendiol erhalten wird. Dibrombutendiol hat zusätzlich den Vorteil, daß es nicht so leicht Halogenwasserstoff abspaltet wie die zuvor genannten aliphatischen Halogenverbindungen. Nach diesem Verfahren wird das Dibrombutendiol in einem Polyol vorgelegt und anschließend unter saurer Katalyse alkoxyliert. Dieses Verfahren hat jedoch mehrere Nachteile. So muß z. B. das Dibrombutendiol in reiner Form hergestellt werden. Die ist zwar durchführbar, jedoch zeigt die Praxis, daß die Bromierung von Butindiol in einem Lösungsmittel, Filtration, Umkristallisation und Trocknung — relativ aufwendig ist. Außerdem muß das so hergestellte Dibrombutendiol für die Alkoxylierung wieder gelöst werden. Dies erschwert die ganze Synthese nicht unerheblich.

Entscheidend ist jedoch auch ein weiterer Nachteil. Durch die Abmischung mit Polyol wird bei der Alkoxylierung nicht nur das Dibrombutendiol, sondern auch das restliche Polyolgemisch umgesetzt, d. h. nach diesem Verfahren liegt immer noch unumgesetztes Dibrombutendiol vor.

Dieses kann zwar zum größten Teil nach der Reaktion abfiltriert werden, die Mengen, die dann jedoch noch im Produkt verbleiben, fallen aber bei Zusatz von — in Hartschaumrezepturen üblichen — Treibmitteln wieder aus und erschweren so einen Einsatz dieser Produkte enorm, wenn sie ihn gar nicht ganz verhindern.

Nach der DE-OS 24 45 571 erhält man halogenierte Polyetherpolyole durch Umsatz von Dibrombutendiol mit Epichlorhydrin. Mit diesen Produkten läßt sich ein guter Flammschutz erzielen. Nachteilig erweist sich hier jedoch die relativ hohe Viskosität der Produkte, die insbesondere bei der Verarbeitung (insbesondere bei der Dosierung) hinderlich ist. Auch ist die Synthese dieser halogenierten Polyetherpolyole sehr aufwendig und damit teuer, da sie ja nicht nur die Schwierigkeiten aufweist, die bei der Herstellung von Alkoxylierungsprodukten des Dibrombutendiols anfallen. Zusätzlich muß hier unter Abspaltung von HCl-Gas ein Chlorhydrin zunächst in ein Oxiran überführt werden, um dieses dann durch alkalische Hydrolyse wieder zur gewünschten hydroxyfunktionellen Verbindung zu öffnen.

Ein anderes Verfahren (JP-PS 78 115 797) zur Bereitstellung bromhaltiger Polyole geht von Butindiol aus, welches unter saurer Katalyse mit Propylenoxid umgesetzt und anschließend mit Brom halogeniert wird. Auch dieser Weg weist in der Praxis mehrere Nachteile auf. So wird bei der Alkoxylierung des Butindiols unter saurer Katalyse bei den angestrebten geringen Molmassen (1 Mol Butindiol + 1,5 bis 2,5 Mol Alkoxyd) nur ca. 75 bis 80 % des Butindiols umgesetzt (siehe Beispiele 4 und 5 bzw. 10 und 11). Ebenso erhält man bei saurer Katalyse ein sehr breites Isomerenspektrum. Bei alkalischer Katalyse liegen dagegen im wesentlichen nur zwei Isomere vor (Beispiel 4 und 5). Bei der nachfolgenden Bromierung wurd nun das freie Butindiol zum 2,3-Dibrombutendiol halogeniert, welches z. T. sofort (Beispiel 10) oder erst bei Zugabe von Frigen[(R)]-Treibmittelzusätzen ausfällt. Insbesondere der letzte Fall steht einer Anwendung dieser Produkte sehr im Wege. Eine unvollständige Umsetzung von Butindiol mit Alkoxyden ließe sich vermeiden, wenn nicht sauer, sondern alkalisch katalysiert würde, wie es in der US-PS 3 366 557 und 3 292 191 beschrieben wird. Die alkalischen Katalysatoren neigen jedoch dazu, eine höhere Oxalkylierungsstufe herbeizuführen und vor allem auch die Rückreaktionen zu katalysieren, d. h. es findet sich im Endprodukt häufig nichtumgesetztes Butindiol bzw. zu hoch alkyliertes Butindiol. Im ersteren Fall führte dies wegen der sehr kritischen Stabilität des Butindiols, unter alkalischen Bedingungen mitunter zu Explosionen und im letzteren Fall wurden die Produkte derart verunreinigt, daß sie auf vielen Gebieten nicht mehr brauchbar waren.

Auch die Verwendung von Aminen als basische Katalysatoren, die ebenfalls in den obengenannten Patentschriften vorgeschlagen werden, konnten diese Nachteile nicht beheben, da diese Variante höhere Temperaturen erfordert, die ihrerseits zu einer Erhöhung der Tendenz zur Bildung von Nebenprodukten und zur erhöhten Bereitschaft des Butindiols zum explosiven Zerfall führt.

Eine andere Entwicklung lehrt die DE-OS 2 036 278. Hier werden als basische Agentien basische Ionenaustauscherharze vorgeschlagen, die zwar hinsichtlich der Selektivität der Reaktion große Fortschritte gebracht haben, jedoch die übrigen Nachteile, wie Rückreaktion und Zersetzungsgefahr, noch nicht auszuschließen vermögen.

Eine weitere Möglichkeit beschriebt die DE-OS 2 241 156, welche als Katalysatoren Thiodialkohole mit niedriger Molmasse, insbesondere Thiodiglykol einsetzt. Nachteil dieses Verfahrens ist, daß Verbindungen wie Thiodiglykol unter den Reaktionsbedingungen zur Bildung leicht flüchtiger ringförmiger Thioether neigen, welche dem Produkt einen so unangenehmen Geruch verleihen, daß eine Anwendung in Frage gestellt ist. Die üblichen Maßnahmen zur Geruchsbeseitigung, wie z. B. Wasserstoffperoxidbehandlung versagen hier, so daß die so katalysierten Verfahrensprodukte einen nicht akzeptablen Geruch aufweisen Außerdem ist Thiodiglykol als Katalysator nicht optimal wirksam. Das Alkoxylierungsprodukt von 1 Mol Butindiol mit 2 Mol Ethylenoxid weist noch relativ viel (sehr störendes) Ausgangsmaterial (Butindiol) auf (s. Beispiel 13).

Es sind also bisher keine Verfahren bekannt, die ein monomerenarmes (Butindiol-1,4-armes) und geruchsfreies Alkoxylierungsprodukt niederer Molmasse aus Butindiol und Alkoxyden liefern. Darüber hinaus fehlt jedoch auch eine sinnvolle Synthese der entsprechenden bromierten Produkte.

Der Einsatz von 2,3-Dibrombutendiol-1,4 als Starterdiol (US-PS 37 64 546) ist aus den bereits genannten Gründen nicht ökonomisch. Auch der Weg, den die JP-PS 78 115 797 lehrt, ist in der beschriebenen Weise nicht anwendbar, da er nicht zu neutralen Produkten führt. So entsteht bei der Bromierung von Alkoxylierungsprodukten von Butindiol zu einem beträchtlichen Anteil Bromwasserstoff aus einer nicht zu unterdrückenden Nebenreaktion. Da dieser Bromwasserstoff jedoch nicht im Produkt verbleiben kann, muß er durch Neutralisation und Filtration entfernt werden. Die erschwert und verteuert die Synthese stark.

Ebenso hat die Erfahrung gezeigt, daß solche Bromierungsprodukte nicht lagerstabil sind. So bemerkt man innerhalb einiger Tage bis Wochen einen deutlichen Anstieg der Säurezahl bzw. ein Absinken des pH-Wertes, was zu einer ständigen Änderung der Verarbeitungsbedingungen führt. Verursacht wird diese pH-Verschiebung durch Spuren von labil gebundenem Brom, welches bei Lagerung langsam als Bromwasserstoff abgespalten wird.

An dem bisher Gesagten erkennt man, daß zwar verschiedene Methoden bekannt sind, bromhaltige Polyole herzustellen, jedoch führt keiner dieser Wege in einer einfachen Synthese zu lagerstabilen, flüssigen bromhaltigen und an 2,3-Dibrombutendiol-1,4-armen Produkten.

Es bestand also die Aufgabe, ein Verfahren zur Herstellung von lagerstabilen, flüssigen, geruchsfreien oder geruchsarmen, bromhaltigen, 2,3-Dibrombutendiol-(1,4)-armen Alkoxylierungsprodukten aus Butindiol-(1,4), Epoxiden und Brom zu finden, welches die genannten Nachteile der bekannten Synthesen nicht aufweist und bromhaltige Polyole zu finden, die sich problemlos bei der Herstellung von flammwidrigen Polyurethanen einsetzen lassen.

Die Aufgabe konnte dadurch gelöst werden, daß man Butindiol-1,4 in Gegenwart bestimmter Katalysatoren (a, b und/oder c, vorzugsweise a und/oder b), mit Epoxiden umsetzt, bei der Bromierung den in einer Nebenreaktion entstehenden Bromwasserstoff durch Oxidation in reaktives Brom überführt und das Endprodukt stabilisiert. Solchermaßen hergestellte Produkte lassen sich problemlos zur Herstellung von flammwidrigen PU-Schaumstoffen einsetzen.

Gegenstand der vorliegenden Erfindung ist somit ein Verfahren zur Herstellung von lagerstabilen, flüssigen, bromhaltigen, 2,3-Dibrombutendiol-(1,4)-armen Alkoxylierungsprodukten aus Butindiol-(1,4), unter katalytischer Anlagerung von Epoxiden und anschließender Bromierung, dadurch gekennzeichnet, daß man

A) Butindiol-(1,4) mit 1,5 bis 2,5 Mol, vorzugsweise mit 1,98 bis 2,05 Mol an Epoxiden, vorzugsweise Ethylenoxid und/oder Propylenoxid, in Gegenwart eines Katalysators aus der Gruppe

a) der höhermolekularen Thioether mit einem MG von 500 bis 5 000, wie sie durch Kondensation, vorzugsweise saurer Kondensation unter Verwendung konzentrierter Phosphorsäure, von Thiodiglykol erhalten werden und/oder

b) von Thioethern, wie sie durch Anlagerung von Mercaptanen und/oder Mercaptoalkoholen, vorzugsweise Thiodiglykol, an ungesättigte Kohlenwasserstoffe erhalten werden, oder ihren Kondensationsprodukten zu höhermolekularen Thioethern mit Molekulargewichten bis 5 000, oder

c) eines Alkalichlorids,

bei erhöhten Temperaturen umsetzt,

B) das entstehende Butindiol-(1,4)-arme Alkoxylierungsprodukt bei — 10 °C bis 80 °C, vorzugsweise bei 0 °C bis 40 °C, besonders bevorzugt bei 0 °C bis 20 °C, mit 0,6 bis 1,9 Mol, vorzugsweise 0,98 bis 1,02 Mol, Brom pro Mol Butindiol umsetzt,

C) den in einer Nebenreaktion entstehenden Bromwasserstoff durch Rückoxidation mit wäßriger Wasserstoffperoxidlösung in reaktives Brom überführt,

D) mit einer schwerflüchtigen Epoxidverbindung, vorzugsweise in Mengen von 0,3 bis 3 Gew.-%, stabilisiert und

E) das bromierte Endprodukt nach Stufe C) oder D) entwässert.

Darüber hinaus betrifft die Erfindung ein Verfahren zur Herstellung von flammwidrigen Polyurethan-Kunststoffen, dadurch gekennzeichnet, daß man diese bromhaltigen Alkoxylierungsprodukte des Butindiols zumindest teilweise, gegebenenfalls unter Zumischung anderer einbaufähiger oder nicht einbaufähiger Flammschutzmittel, bei der üblichen Herstellung von PU-Schaumstoffen nach dem Polyisocyanat-Polyadditionsverfahren verwendet.

Die Umsetzung von Butindiol-(1,4) mit 1,5 bis 2,5 Mol Epoxiden kann, da sie zu Molekülen mit relativ niedriger Molmasse führt, nicht sauer — z. B. mit $BF_3$ — katalysiert werden. Unter solchen Bedingungen bleiben 15 bis 20 % des eingesetzten Butindiols im Reaktionsgemisch zurück (vgl. Beispiele 4 bis 10), welches bei der anschließenden Bromierung aus o. a. Gründen stören würde (Beispiel 4 und 10).

Erfindungsgemäß wird die Umsetzung von Butindiol mit Epoxiden unter Katalyse spezieller Verbindungen durchgeführt.

Als erste Gruppe a) von Katalysatoren sind hier die Polythioether mit einem MG von 500 bis 5 000 zu nennen, welche durch Kondensation von Thiodiglykol erhalten werden können. Diese höhermolekularen bzw. kettenförmigen Polythioether, deren Herstellung und Eigenschaften in der DE-AS 1 039 232 beschrieben sind, zeigen gegenüber den niedermolekularen Thiodiglykolen der DE-OS 2 241 156 den wesentlichen Vorteil, daß der den Produkten anhaftende Geruch durch Oxidation mit Wasserstoffperoxid in der Stufe c) entfernt werden kann. Bei Verwendung von niedermolekularen Thioethern ist dies dagegen nicht oder nur unvollständig möglich. Außerdem liegt bei Verwendung der erfindungsgemäßen Katalysatoren nach der Alkoxylierung wesentlich weniger freies Butindiol-1,4 vor (s. Beispiel 2 und 13).

Eine weitere Gruppe b) von Katalysatoren erhält man durch Anlagerung von Mercaptoalkanen, insbesondere Mercaptoethanol an Verbindungen, welche Doppelbindungen aufweisen. Diese Verbindungen besitzen zumeist keinen Thioether-üblichen, unangenehmen Geruch, der einen Einsatz verhindern würde. Als erfindungsgemäß besonders geeignet erweist sich das Umsetzungsprodukt aus 2 Mol Mercaptoethanol und Limonen (1-Methyl-2-(2-hydroxy-ethylthio)-4-[1-methyl-2-(2-hydroxyethylthio)ethyl] cyclohexan). Entsprechend Thiodiglykol kann natürlich auch diese Verbindung zu höhermolekularen Einheiten, vorzugsweise mit Molekulargewichten bis zu 5 000 kondensiert werden.

Weiterhin lassen sich erfindungsgemäß als Katalysatoren für die Reaktion von Butindiol-(1,4) mit Epoxiden auch Alkalichloride c), insbesondere Kaliumchlorid verwenden. Diese besitzen ebenso die geforderten katalytischen Eigenschaften, d. h. man erhält bei ihrem Einsatz einen praktisch vollständigen Umsatz des Butindiols, und das resultierende Produkt weist keinen unangenehmen Geruch auf. Der Nachteil der Alkalichloride ist jedoch, daß sie aus dem Reaktionsgemisch durch Filtration entfernt werden müssen. Aus diesem Grunde werden die angeführten Thioether a) bzw. b) erfindungsgemäß bevorzugt.

Die Thioether der Gruppe a) lassen sich in einfacher Weise durch Kondensation von Thiodiglykol erhalten. Hier hat sich insbesondere die saure Veretherung mit Phosphorsäure bzw. Phosphorsäuraderivate als vorteilhaft erwiesen, da hierbei keine Thioxane bzw. Dithiane entstehen, die dem Produkt einen sehr unangenehmen Geruch verleihen (DE-AS 1 039 232). Der Veretherungsgrad läßt sich leicht über die Reaktionsdauer bzw. über die abgespaltene Wassermenge steuern.

Die Thioether der Gruppe b) werden durch radikalische Addition von Mercaptanen oder Mercaptoethanolen an Doppelbindungen hergestellt. Abweichend von bekannten Synthesen (US-PS 4 013 724) werden jedoch keine Radikalstarter wie z. B. Azobisisobutyronitril (AIBN), Benzoylperoxid usw., die erst bei erhöhter Temperatur wirksam werden, eingesetzt. In einfacher Weise wird dagegen die Radikalbildende Wirkung von Luftsauerstoff genutzt, um die gewünschten Thioether aus den Mercaptanen bzw. Mercapto-ethanolen und ungesättigten Systemen zu synthetisieren. (s. Beispiel zur Katalysatorherstellung). Die Kondensation zu höhermolekularen Einheiten erfolgt entsprechend den Verfahren der Herstellung der Katalysatorgruppe a).

Die Alkoxylierung des Butindiols mit 1,5 bis 2,5 Mol Epoxid wird bei erhöhter Temperatur und erfindungsgemäß unter Zusatz katalytisch wirksamer Mengen der angeführten Katalysatoren, z. B. 0,1 bis 3 Gew.-%, bevorzugt mit 0,1 bis 1 Gew.-%, bezogen auf das Gesamtgewicht, durchgeführt.

Die so erhaltenen Alkoxylierungsprodukte werden zur weiteren Umsetzung mit Brom bei — 10 °C bis 80 °C, vorzugsweise bei 0 °C bis 40 °C und besonders bevorzugt bei + 5 °C bis 25 °C umgesetzt.

Pro Mol Butindiol werden 0,6 bis 1,9 Mol, vorzugsweise 0,98 bis 1,02 Mol Brom eingesetzt. Die Anlagerung des Broms an die Dreifachbindung, wie sie auch in der JP-PS 78 115 797 beschrieben wird, ist jedoch nicht vollständig. Da zu einem nicht unbeträchtlichen Teil (ca. 10 %) in nicht zu unterdrückenden Nebenreaktionen Bromwasserstoff entsteht, lassen sich so erhaltene Produkte nicht ohne größeren Aufwand, d. h. Neutralisation und Filtration, einsetzen. Dies bedeutet im übrigen nicht nur eine Verteuerung der Synthese, sondern auch (durch den relativ hohen Salzanfall aus der Neutralisation) eine zusätzliche Umweltbelastung.

Daher wird im erfindungsgemäßen Verfahren die Bromierung wesentlich vereinfacht. So wird der in der Bromaddition in Stufe B) anfallende Bromwasserstoff durch Zugabe etwa äquivalenter Mengen oder leicht überschüssiger Menge (ca. 15 % Überschuß) Wasserstoffperoxids zu Brom rückoxidiert. Da im Reaktionsgemisch noch genügend Dreifachbindungen des Butindiols vorhanden sind, lagert sich dieses Brom sofort an diese an, d. h. der entstandene Bromwasserstoff geht der Synthese nicht verloren, sondern wird gleichfalls als Brom zur Vervollständigung der Additionsreaktion an die Dreifachbindung gebracht. Die Temperatur liegt vorzugsweise bei 0 °C bis 40 °C, vorzugsweise 5 °C bis 25 °C.

Diese Verfahrensweise, d. h. in situ Herstellung von Brom durch Aufoxidation von HBr mit Oxidationsmitteln, ist zwar prinzipiell bekannt (DE-OS 3 133 577), jedoch wurde sie bisher nur an reinem Butindiol erprobt. Überraschenderweise gelingt diese Oxidation des Bromwasserstoffs jedoch auch bei Anwesenheit der alkoxylierten Butindiole, ohne eine Spaltung an der Etherbindung herbeizuführen.

Das bei der Reaktion des Wasserstoffperoxids mit HBr entstehende Wasser kann leicht im Vakuum entfernt werden. Die Entwässerungsstufe E) kann aber auch nach dem Epoxidzusatz (D) erfolgen.

Weiter hatte sich bei den bisher bekannten Bromierungen gezeigt, daß die Produkte nicht lagerstabil sind. Es liegen also nach der Bromierung noch geringe Mengen von labil gebundenem Brom vor, welche nach einigen Tagen bzw. Wochen als Bromwasserstoff abgespalten werden. Dies führt dazu, daß solche Produkte nach einem gewissen Zeitraum nicht mehr eingesetzt werden können, da die erhöhte Säurezahl die Reaktivität verändert und z. B. auch die Amin-Katalyse bei der Herstellung von Polyurethanschaumstoffen in nicht vorhersehender Weise stört.

Erfindungsgemäß wird dieser Anstieg an freiem Bromwasserstoff bzw. die Erhöhung der Säurezahl bei Lagerung dadurch verhindert, daß zur Stabilisierung schwerflüchtige Epoxidverbindungen zugesetzt werden (Stufe D). Diese Epoxidverbindungen können beispielsweise durch Umsetzung von Epihalohydrinen, wie Epichlorhydrin, mit polyfunktionellen Phenolen, wie z. B. 2,2-Bis-(4-hydroxyphenyl)-propan, 1-(4-Hydroxyphenyl)-1-(2-hydroxyphenyl)-propan, 3,3-Bis-(3-hydroxyphenyl)-pentan, 2-(3-Hydroxyphenyl)-2-(4-hydroxyphenyl)-pentan, 1-(2-Hydroxyphenyl)-1-(3-hydroxyphenyl)-1-phenyl-propan, 1,1-Bis-(4-hydroxyphenyl)-1-phenyl-butan, Hydrochinon, alkylierten Hydrochinonen oder Alkoholen wie Ethylenglykol, Propylenglykol, Hexandiol, Pentaerythrit oder auch deren höheren Homologen, wie Polyethylenglykol und Polypropylenglykol, gewonnen werden.

Solche Epoxidverbindungen haben den Vorteil, daß sie einerseits schnell mit vorhandenen HBr-Spuren zu den entsprechenden Bromhydrinen abreagieren und andererseits durch ihre Schwerflüchtigkeit lange im Produkt verbleiben. Die schwerflüchtigen Epoxidverbindungen, wie z. B. Levepox 4020[R], Produkt und eingetragenes Warenzeichen der BAYER AG, D 5090 Leverkusen, werden erfindungsgemäß in Mengen von 0,3 bis 3 Gew.-%, bezogen auf Gesamtgewicht, eingesetzt. Die Molekulargewichte solcher schwerflüchtiger Epoxide liegen zumeist oberhalb von 174, vorzugsweise oberhalb von 300.

Die nach den beschriebenen Verfahren hergestellten bromierten Alkoxylierungsprodukte des Butindiols haben den Vorteil, daß sie zu lagerstabilen, flüssigen, geruchsfreien oder geruchsarmen 2,3-Dibrombutendiol-(1,4)-armen Produkten führen, die sich aufgrund ihrer einfachen Herstellung und Handhabbarkeit hervorragend zur Herstellung von flammwidrigen PU-Kunststoffen eignen.

Gegenstand der vorliegenden Erfindung ist demzufolge auch die Verwendung der bromhaltigen Alkoxylierungsprodukte in einem Verfahren zur Herstellung von flammwidrigen Kunststoffen auf Polyisocyanatbasis, vorzugsweise flammwidrigen PU-Schaumstoffen, einschließlich Polyisocyanurat-Schaumstoffen, durch Umsetzung von organischen Polyisocyanaten mit höhermolekularen Verbindungen mit gegenüber NCO reaktiven Gruppen mit reaktiven H-Atomen, z. B. OH—, NH$_2$—, NHR—, COOH und/oder SH-Gruppen, vorzugsweise höhermolekularen Polyhydroxylverbindungen, gegebenenfalls Wasser und/oder niedermolekularen Kettenverlängerungsmitteln oder Vernetzern in Gegenwart der in der PU-Chemie üblichen Hilfs- und Zusatzstoffe, Katalysatoren, einschließlich Trimerisierungskatalysatoren, dadurch gekennzeichnet, daß man als höhermolekulare Polyhydroxylverbindung zumindest teilweise in Mengen von 1 bis 60 Gew.-%, die nach o. a. Verfahren hergestellten bromhaltigen Alkoxylierungsprodukte, gegebenenfalls unter Zumischung anderer einbaufähiger oder nicht einbaufähiger Flammschutzmittel, einsetzt.

Hierzu werden vom erfindungsgemäßen bromhaltigen Polyol 1 bis 60 Gew.-%, bevorzugt 3 bis 50 Gew.-%, insbesondere 5 bis 40 Gew.-%, in die höhermolekulare Polyolkomponente eingebracht.

Selbstverständlich kann aber auch zuerst mit der Isocyanatkomponente ein sogenanntes Prepolymer angefertigt werden, das zu weiteren Reaktionen verwendet wird.

Zur Herstellung der Polyurethan(harnstoff)-Schaumstoffe wird üblicherweise in einem NCO-Kennzahlbereich von 100 bis 135 gearbeitet, jedoch kann der Kennzahlbereich auch oberhalb von 135 (z. B. bis 500) liegen, insbesondere wenn man mit Trimerisierungskatalysatoren für NCO isocyanuratgruppenhaltige PU-Schaumstoffe (Vorzugs-Kennzahlbereich 135 bis 500) herstellt.

Als Flammschutzmittel können zusätzlich einbaufähige oder nicht einbaufähige Flammschutzmittel mitverwendet werden. Diese Flammschutzmittel sind an sich bekannt, beispielsweise seien genannt: Trichlorethylphosphat, Trichlorpropylphosphat, Diphenylkresylphosphat, Trikresylphosphat, halogenierte Kohlenwasserstoffe und halogenierte Aromaten, wie Dekabromdiphenylether oder auch Ammoniumphosphate und -polyphosphate, Phosphonsäureester, wie Methylphosphonsäuredimethylester oder Ester, wie sie z. B. nach der DOS 2 750 555 erhalten werden oder Phosphorsäureester, wie sie nach der DE-AS 1 181 411 bekannt geworden sind. Als einbaufähige Flammschutzmittel können auch Amine entsprechend der DE-OS 2 406 163 eingesetzt werden. Bei Verwendung werden vorzugsweise neben den erfindungsgemäß einzusetzenden bromhaltigen Alkoxylierungsprodukten zusätzlich 3 bis 25 Gew.-%, bezogen auf Gesamtgewicht, phosphorhaltiger und/oder halogen-, insbesondere bromhaltiger, einbaufähiger oder nicht einbaufähiger Flammschutzmittel mitverwendet.

Die Herstellung von Kunststoffen auf Isocyanatbasis ist an sich bekannt und z. B. in den deutschen Offenlegungsschriften 1 694 142, 1 694 215 und 1 720 768 sowie im Kunststoff-Handbuch Band VII, Polyurethane, herausgegeben von Vieweg und Höchtlen, Carl Hanser Verlag München 1966 sowie in der Neuauflage dieses Buches, herausgegeben von G. Oertel, Carl Hanser Verlag München, Wien 1983, beschrieben.

Es handelt sich dabei vorwiegend um Urethan- und/oder Isocyanurat- und/oder Allophanat- und/oder Uretdion- und/oder Harnstoff- und/oder Carbodiimidgruppen aufweisende Kunststoffe. Die erfindungsgemäße Verwendung erfolgt vorzugsweise bei der Herstellung von Polyurethan- und Polyisocyanuratkunststoffen, insbesondere Schaumstoffen.

Ausgangsstoffe für die Herstellung der Kunststoffe auf Isocyanatbasis

Als Isocyanatkomponente, die als niedermolekulare di- oder mehrfunktionelle Isocyanate oder zur Herstellung der nieder- oder höhermolekularen, Isocyanatgruppen-haltigen Prepolymeren eingesetzt werden können, kommen aliphatische, cycloaliphatische, araliphatische, aromatische und/oder heterocyclische Polyisocyanate in Betracht, wie sie aus einer Vielzahl von Veröffentlichungen für die Verwendung zum Aufbau von Polyurethan bekannt sind und beispielsweise von W. Siefken in Justus Liebigs Annalen der Chemie 562, Seiten 75 bis 136, 1949, und beispielsweise in den DE-OS 2 854 384 und 2 920 501 beschrieben wurden. Beispiele sind: Ethylen-diisocyanat, 1,4-Tetramethylendiisocyanat, 1,6-Hexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanatomethyl-cyclohexan, 2,4- und 2,6-Hexahydrotoluylendiisocyanat sowie beliebige Gemische dieser Isomeren, Hexahydro-1,3- und/oder -1,4-phenylen-diisocyanat, Perhydro-2,4'- und/oder -4,4'-diphenylmethan-diisocyanat, 1,3- und 1,4-Phenylen-diisocyanat, 2,4- und 2,6-Toluylendiisocyanat sowie beliebige Gemische dieser Isomeren, Diphenylmethan-2,4'-, 2,2'- und/oder 4,4'-diisocyanat, Naphthylen-1,5-diisocyanat, Triphenylmethan-4,4',4''-triisocyanat, Polyphenyl-polymethylen-polyisocyanate, wie sie durch Anilin-Formaldehyd-Kondensation und anschließende Phosgenierung erhalten und z. B. in den britischen Patentschriften 874 430 und 848 671 beschrieben werden, perchlorierte Arylpolyisocyanate, wie sie z. B. in der DE-AS 1 157 601 beschrieben werden, Carbodiimidgruppen aufweisende Polyisocyanate, wie sie in der DE-PS 1 092 007 beschrieben werden, Diisocyanate, wie sie in der US-PS 3 492 330 beschrieben werden, Allophanatgruppen aufweisende Polyisocyanate, wie sie z. B. in der GB-PS 994 890, der BE-PS 761 626 und der veröffentlichten holländischen Patentanmeldung 7 102 524 beschrieben werden, Isocyanuratgruppen aufweisende Polyisocyariate, wie sie z. B. in den DE-PSen 1 022 789, 1 222 067 und 1 027 394 sowie in den DE-OSen 1 929 034 und 2 004 048 beschrieben werden, Urethangruppen aufweisende Polyisocyanate, wie sie z. B. in der BE-PS 752 261 oder in der US-PS 3 394 164 beschrieben werden, acylierte Harnstoffgruppen aufweisende Polyisocyanate gemäß der DE-PS 1 230 778, Biuretgruppen aufweisende Polyisocyanate, wie sie z. B. in der DE-PS 1 101 394, in der GB-PS 889 050 und in der FR-PS 7 017 514 beschrieben werden, durch Telomerisationsreaktionen hergestellte Polyisocyanate, wie sie z. B. in der BE-PS 723 640 beschrieben werden, Estergruppen aufweisende Polyisocyanate, wie sie z. B. in den GB-PSen 965 474 und 1 072 956, in der US-PS 3 567 763 und in der DE-PS 1 231 688 genannt werden, sowie Umsetzungsprodukte der obengenannten Isocyanate mit Acetalen gemäß der DE-PS 1 072 385. Ferner ist es möglich, beliebige Mischungen dieser Polyisocyanate zu verwenden.

Besonders bevorzugt werden in der Regel die technisch leicht zugänglichen Polyisocyanate, z. B. das 2,4- und 2,6-Toluylendiisocyanat sowie beliebige Gemische dieser Isomeren (« TDI »), Polyphenyl-polymethylen-polyisocyanate, wie sie durch Anilin-Formaldehyd-Kondensation und anschließende Phosgenierung hergestellt werden (« rohes MDI »), 4,4'- und/oder 2,4'-Diphenylmethan-4,4'-diisocyanat und Carbodiimidgruppen, Urethangruppen, Allophanatgruppen, Isocyanuratgruppen, Harnstoffgruppen oder Biuretgruppen aufweisende Polyisocyanate (« modifizierte Polyisocyanate »).

Ebenso können die Polyisocyanate auch in einer stabilisierten Form mit retardierter Reaktivität, entsprechend den Verfahren nach den DE-OSen 3 112 054, 3 228 723, 3 228 724, 3 228 670 und 3 230 757 und der EP-A 2 230 757 eingesetzt werden.

Als gegebenenfalls zu verwendende Ausgangskomponenten werden ferner höhermolekulare Verbindungen mit mindestens zwei Gruppen mit gegenüber Isocyanaten reaktionsfähigen Wasserstoffatomen, von einem Molekulargewicht in der Regel von 400-10 000 verwendet. Hierunter versteht man Hydroxyl/ Aminogruppen, Thiolgruppen oder Carboxylgruppen aufweisenden Verbindungen. Vorzugsweise handelt es sich dabei um Hydroxylgruppen aufweisende Verbindungen, insbesondere zwei bis acht Hydroxylgruppen aufweisende Verbindungen, speziell solche vom Molekulargewicht 1 000 bis 5 000, vorzugsweise 800 bis 3 000. Es sind dies z. B. mindestens zwei, in der Regel 2 bis 8, vorzugsweise aber 2 bis 4, Hydroxylgruppen aufweisende Polyester, Polyether, Polythioether, Polyacetale, Polycarbonate· und Polyesteramide sowie andere höhermolekulare Verbindungen wie sie für die Herstellung von homogenen und von zellförmigen Polyurethanen an sich bekannt sind. Beispiele hierfür sind in der DE-OS 29 20 501 und der DE-OS 2 854 384 aufgeführt.

Die in Frage kommenden Hydroxylgruppen aufweisenden Polyester sind z. B. Umsetzungsprodukte von mehrwertigen, vorzugsweise zweiwertigen und gegebenenfalls zusätzlich dreiwertigen Alkoholen mit mehrwertigen, vorzugsweise zweiwertigen, Carbonsäuren, Polycarbonsäureanhydriden oder Polycarbonsäureestern von niedrigen Alkoholen. Die Polycarbonsäuren können aliphatischer, cycloaliphatischer, aromatischer und/oder heterocyclischer Natur sein und gegebenenfalls, z. B. durch Halogenatome, substituiert und/oder ungesättigt sein. Als Beispiele hierfür seien genannt : Bernsteinsäure, Adipinsäure, Korksäure, Azelainsäure, Sebacinsäure, Phthalsäure, Isophthalsäure, Trimellitsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, Ölsäure, dimere und trimere Fettsäuren, gegebenenfalls in Mischung mit monomeren Fettsäuren, Terephthalsäuredimethylester und Terephthalsäure-bis-glykolester. Als mehrwertige Alkohole kommen z. B. Ethylenglykol, Propandiol-(1,2) und -(1,3), Butandiol-(1,4) und -(2,3), Hexandiol-(1,6), Octandiol-(1,8), Neopentylglykol, 1,4-Bis-hydroxymethylcyclohexan, 2-Methyl-1,3-propandiol, Glycerin, Trimethylolpropan, Hexantriol-(1,2,6), Butantriol-(1,2,4), Trimethylolethan, Pentaerythrit, Chinit, Mannit und Sorbit, 1,4,3,6-Dianhydrohexite, Methylglykosid, ferner Di-, Tri-, Tetra- und Polyethylenglykol, Di-, Tri-, Tetra- und Polypropylenglykol, Di-, Tri-, Tetra- und Polybutylenglykol in Frage. Die Polyester können anteilig endständige Carboxylgruppen aufweisen. Auch Polyester aus Lactonen, z. B. ε-Caprolacton oder Hydroxycarbonsäuren, z. B. ω-Hydroxycapronsäure, sind einsetzbar.

Auch die erfindungsgemäß in Frage kommenden, mindestens zwei, in der Regel zwei bis acht, vorzugsweise zwei bis drei, Hydroxylgruppen aufweisenden Polyether sind solche der an sich bekannten Art und werden z. B. durch Polymerisation von Tetrahydrofuran und/oder Epoxiden wie Ethylenoxid, Propylenoxid, Butylenoxid, Styroloxid oder Epichlorhydrin mit sich selbst, z. B. in Gegenwart von $BF_3$, oder durch Anlagerung dieser Epoxide, gegebenenfalls im Gemisch oder nacheinander, an Startkomponenten mit reaktionsfähigen Wasserstoffatomen wie Wasser, Alkohole, Aminoalkohole, Amine, Zucker u. a. bekannte Startkomponenten, z. B. Ethylenglykol, Propandiol-(1,3) oder -(1,2), Trimethylolpropan, 4,4'-Dihydroxydiphenylpropan, Anilin, Ammoniak, Ethanolamin oder Ethylendiamin hergestellt. Auch Sucrosepolyether, wie sie z. B. in den DE-AS 1 176 358 und 1 064 938 beschrieben werden, kommen erfindungsgemäß in Frage. Beispiele sind höhermolekulare Polyoxyalkylen-polyole, z. B. Polyoxytetra-

7

methylenglykole oder Ethoxylierungs- und/oder Propoxylierungsprodukte von niedermolekularen Di- und Polyolen oder Mono-, Di- und Polyaminen, z. B. propoxyliertes Trimethylolpropan, propoxyliertes Ethylendiamin oder lineare oder verzweigte Polypropylenglykolether, die anteilsweise in statistischer, blockartiger oder endständiger Form Ethylenoxid enthalten können und insgesamt Molekulargewichte von 400 bis 10 000, vorzugsweise 600 bis 6 000, aufweisen. Auch durch Vinylpolymerisate modifizierte Polyether, wie sie z. B. durch Polymerisation von Styrol und Acrylnitril in Gegenwart von Polyethern entstehen (US-PSen 3 383 351, 3 304 273, 3 523 093, 3 110 695, DE-PS 1 152 536), sind geeignet, ebenso OH-Gruppen aufweisende Polybutadiene. Polyether mit sekundären OH-Gruppen sind als Suspendiermedium bevorzugt.

Unter den Polythioethern seien insbesondere die Kondensationsprodukte von Thiodiglykol mit sich selbst und/oder mit anderen Glykolen, Dicarbonsäuren, Formaldehyd, Aminocarbonsäuren oder Aminoalkoholen angeführt.

Als Polyacetale kommen z. B. die aus Glykolen, wie Diethylenglykol, Triethylenglykol, 4,4'-Dioxethoxy-diphenyldimethylmethan, Hexandiol und Formaldehyd herstellbaren Verbindungen in Frage. Auch durch Polymerisation cyclischer Acetale lassen sich erfindungsgemäß geeignete Polyacetale herstellen.

Als Hydroxylgruppen aufweisende Polycarbonate kommen solche der an sich bekannten Art in Betracht, die z. B. durch Umsetzung von Diolen wie Propandiol-(1,3), Butandiol-(1,4) und/oder Hexandiol-(1,6), Di-, Tri- oder Tetraethylenglykol mit Diarylcarbonaten, z. B. Diphenylcarbonat, oder mit Phosgen hergestellt werden können.

Zu den Polyesteramiden und Polyamiden zählen z. B. die aus mehrwertigen gesättigten und ungesättigten Carbonsäuren bzw. deren Anhydriden und mehrwertigen gesättigten und ungesättigten Aminoalkoholen, Diaminen, Polyaminen und ihren Mischungen gewonnenen, vorwiegend linearen Kondensate.

Auch bereits Urethan- oder Harnstoffgruppen enthaltende Polyhydroxylverbindungen sowie gegebenenfalls modifizierte, natürliche Polyole, wie Rizinusöl, Kohlenhydrate oder Stärke, sind verwendbar. Auch Anlagerungsprodukte von Alkylenoxiden an Phenol-Formaldehyd-Harze bzw. an Harnstoff-Formaldehydharze können im erfindungsgemäßen Verfahren eingesetzt werden.

Auch Hydroxylendgruppen aufweisende Polybutadiene sind erfindungsgemäß geeignet, da sie besonders elastische und hydrolysenstabile Produkte ergeben. Es können gegebenenfalls auch Polyhydroxylverbindungen eingesetzt werden, in welchen hochmolekulare Polyaddukte bzw. Polykondensate oder Polymerisate in feindisperser oder auch gelöster Form enthalten sind.

Solche Polyhydroxylverbindungen werden z. B. erhalten, wenn man Polyadditionsreaktionen (z. B. Umsetzungen zwischen Polyisocyanaten und aminofunktionellen Verbindungen) bzw. Polykondensationsreaktionen (z. B. zwischen Formaldehyd und Phenolen und/oder Aminen) in situ in den oben genannten, Hydroxylgruppen aufweisenden Verbindungen ablaufen läßt. Derartige Verfahren sind beispielsweise in den DE-AS 1 168 075 und 1 260 142, sowie den DE-OS 2 324 134, 2 423 984, 2 512 385, 2 513 815, 2 550 796, 2 550 797, 2 550 833, 2 550 862, 2 633 293 und 2 639 254, sowie US-PS 3 869 413 bzw. DE-OS 2 550 860 beschrieben.

Auch durch Vinylpolymerisate modifizierte Polyhydroxylverbindungen, wie sie z. B. durch Polymerisation von Styrol und Acrylnitril in Gegenwart von Polyethern (US-PS 3 383 341, 3 304 273, 3 523 093, 3 110 695 ; DE-AS 1 152 536) oder Polycarbonatpolyolen (DE-PS 1 769 795 ; US-PS 3 637 909) erhalten werden, sind für das erfindungsgemäße Verfahren geeignet. Bei Verwendung von Polyetherpolyolen, welche gemäß den DE-OS 2 442 101, 2 644 922 und 2 646 141 durch Pfropfpolymerisation mit Vinylphosphonsäureestern sowie gegebenenfalls (Meth)acrylnitril, (Meth)acrylamid oder OH-funktionellen (Meth)acrylsäureestern modifiziert wurden, erhält man Kunststoffe von besonderer Flammwidrigkeit. Polyhydroxylverbindungen, in welche durch radikalische Pfropfpolymerisation mittels ungesättigter Carbonsäuren sowie gegebenenfalls weiterer olefinisch ungesättigter Monomerer Carboxylgruppen eingeführt wurden (DE-OS 2 714 291, 2 739 620 und 2 654 746) können mit besonderem Vorteil in Kombination mit mineralischen Füllstoffen eingesetzt werden.

Bei der Verwendung von modifizierten Polyhydroxylverbindungen der oben genannten Art als Ausgangskomponente im Polyisocyanat-Polyadditionsverfahren entstehen in vielen Fällen Polyurethankunststoffe mit wesentlich verbesserten mechanischen Eigenschaften.

Vertreter dieser erfindungsgemäß zu verwendenden Verbindungen zind z. B. in High Polymers, Vol. XVI, « Polyurethanes, Chemistry and Technology », verfaßt von Saunders Frisch, Interscience Publishers, New York, London, Band I, 1962, Seiten 32-42 und Seiten 44 und 54 und Band II, 1964, Seiten 5-6 und 198-199, sowie im Kunststoff-Handbuch, Band VII, Vieweg-Höchtlen, Carl-Hanser-Verlag, München, 1966, z. B. auf den Seiten 45 bis 71, und in der DE-OS 2 854 384 und 2 920 501 beschrieben.

Selbstverständlich können Mischungen der obengenannten Verbindungen mit mindestens zwei gegenüber Isocyanaten reaktionsfähigen Wasserstoffatomen mit einem Molekulargewicht von 400-10 000, z. B. Mischungen von Polyethern und Polyestern, eingesetzt werden.

Als höhermolekulare Verbindungen mit genenüber NCO reaktiven H-Atomen können auch niedrigschmelzende (< 60 °C), höhermolekulare Polyamine mit aromatischen und/oder aliphatischen Aminogruppen und Molekulargewichten von 400 bis 10 000, vorzugsweise 500 bis 6 000 eingesetzt werden.

Als höhermolekulare Polyaminoverbindungen mit aromatischen Aminogruppen mit einem Molekulargewichtsbereich von 400 bis 10 000, vorzugsweise 500 bis 6 000, werden insbesondere solche Polyaminoverbindungen eingesetzt, wie sie durch Hydrolyse von entsprechenden NCO-Prepolymeren auf der Basis von höhermolekularen Polyhydroxylverbindungen und überschüssigen aromatischen Diisocyanaten durch (vorzugsweise basische) Hydrolyse hergestellt werden können. Beispiele für dieses Verfahren werden in der DE-OS 2 948 419, DE-OS 3 039 600, DE-OS 3 112 118, der EP-A 61 627, EP-A 71 132 und EP-A 71 139 angegeben. In der erstgenannten Patentschrift werden auch weitere Verfahren des Standes der Technik zur Herstellung von aromatischen Aminoverbindungen höhermolekularer Struktur genannt, wie sie für das erfindungsgemäße Verfahren geeignet sind. Es handelt sich bei dem Verfahren nach DE-OS 2 948 419 und den anderen zitierten Patentschriften vorzugsweise um Polyether-Polyamine, aber auch Polyester-, Polyacetal-, Polythioether- oder Polycaprolacton-Polyamine, vorzugsweise 2- oder 3-funktionelle Polyamine, welche Urethangruppen (aus der Reaktion der entsprechenden höhermolekularen Polyhydroxylverbindungen mit den überschüssigen Polyisocyanaten) enthalten und die Aminogruppen an dem Rest des (ehemaligen) Polyisocyanats tragen. Die aromatischen, höhermolekularen Polyamine können jedoch auch nach anderen Verfahren, z. B. durch Umsetzung von NCO-Prepolymeren mit überschüssigen Mengen an Aminophenylethylamin, oder anderen Diaminen entsprechend DE-AS 1 694 152 hergestellt werden ; eine andere Synthesemöglichkeit beschreibt die FR-PS 1 415 317 durch Überführung der NCO-Prepolymere mit Ameisensäure in die N-Formylderivate und deren Verseifung. Auch die Umsetzung von NCO-Prepolymeren mit Sulfaminsäure gemäß DE-AS 1 155 907 führt zu höhermolekularen Polyaminen.

Neben an aromatische Reste gebundene Aminogruppen tragenden höhermolekularen Polyaminverbindungen (aus aromatischen Polyisocyanaten) lassen sich auch (über aliphatische Polyisocyanate) an aliphatische Reste gebundene, Aminogruppen tragende höhermolekulare Polyaminoverbindungen herstellen.

Ebenfalls einsetzbar sind höhermolekulare aliphatische Di- und Polyamine, wie sie z. B. durch reduktive Aminierung von Polyoxyalkylenglykolen mit Ammoniak nach BE-PS 634 741 oder US-PS 3 654 370 erhalten werden können. Weitere höhermolekulare Polyoxyalkylen-Polyamine können nach Methoden, wie sie in der Firmenschrift « Jeffamine, Polyoxypropylene Amines » von Texaco Chemical Co., 1978, aufgezählt werden, hergestellt werden, beispielsweise durch Hydrierung von cyanethylierten Polyoxypropylenglykolen (DE-OS 1 193 671), durch Aminierung von Polypropylenglykolsulfonsäureestern (US-PS 3 236 895), durch Behandlung eines Polyoxyalkylenglykols mit Epichlorhydrin und einem primären Amin (FR-PS 1 466 708) oder durch Umsetzung von NCO-Prepolymeren mit Hydroxylgruppen aufweisenden Enaminen, Aldiminen oder Ketiminen und anschließende Hydrolyse gemäß DE-OS 2 546 536. Geeignete höhermolekulare aliphatische Di- und Polyamine sind auch die nach DE-OS 29 48 419 und DE-OS 3 039 600 durch alkalische Hydrolyse von NCO-Prepolymeren (mit aliphatischen Diisocyanaten) mit Basen über die Carbamatstufe zugänglichen Polyamine. Diese höhermolekularen Polyamine besitzen Molekulargewichte von etwa 400 bis 10 000, vorzugsweise 500 bis 6 000 und besonders bevorzugt von 1 000 bis 3 000.

Die erfindungsgemäßen Polyurethankunststoffe werden gegebenenfalls unter Mitverwendung von weiteren niedermolekularen Kettenverlängerern oder Vernetzern hergestellt. Bei diesen niedermolekularen Kettenverlängerern oder Vernetzern handelt es sich um zwei- oder mehrfunktionelle Verbindungen, welche an aliphatische und/oder cycloaliphatische Gruppen gebundene Hydroxylgruppen (Polyole) und/oder an aromatische, einschließlich heterocyclische Ringe mit aromatischem Charakter gebundene NH$_2$-Gruppen (Polyamine) und Molekulargewichte zwischen 62 und 399 aufweisen. Bevorzugt sind dabei niedermolekulare Diole mit an aliphatische oder cycloaliphatische Gruppen gebundenen Hydroxylgruppen, sowie aromatische Diamine des Molekulargewichtsbereichs 108 bis 399.

Diese Verbindungen weisen in der Regel 2 bis 8, vorzugsweise 2 bis 4, besonders bevorzugt jedoch 2, gegenüber Isocyanaten reaktionsfähige Wasserstoffatome wie Hydroxyl- und/oder Aminogruppen auf. Es können selbstverständlich auch Mischungen von verschiedenen Verbindungen verwendet werden. Als Beispiele für derartige Verbindungen seien genannt : Ethylenglykol, Trimethylenglykol, Butandiol-2,3 und/oder -1,4, Hexandiol-1,6, Neopentylglykol, 1,4-Bis-hydroxyethyl-cyclohexan, 1,4-Dihydroxycyclohexan, Terephthalsäure-bis(β -hydroxyethyl) ester, 1,4,3,6-Dianhydrohexite, 1,4-Monoanhydrotetrite, sowie weniger bevorzugt Diole mit sekundären Hydroxylgruppen, z. B. Propylenglykol, Butandiol-2,3, oder Pentandiol-2,5. Als mehrwertige Verbindungen seien genannt : Trimethylolpropan, Trimethylolethan, Hexantriol-1,2,6, Glycerin, Pentaerythrit, Chinit, Mannit, Sorbit, Rizinusöl, sowie Di-, Tri- und Tetraethylen-, -propylen-, und -butylen-glykole, ferner Bis-(2-hydroxyethyl)-hydrochinon, Bis-(2-hydroxyethyl)-resorcin, Formose oder Formit. Ferner sind geeignet tertiäraminhaltige Di- oder Polyole, z. B. N-Methyldiethanolamin, Triethanolamin oder N,N'-Bis-hydroxyethylpiperazin.

Es können aber auch Polyamine, vorzugsweise Diamine, eingesetzt werden.

Unter aromatischen Polyaminen sollen auch solche Amine verstanden werden welche die Aminogruppe an heterocyclische Reste mit aromatischem Charakter gebunden enthalten. Als aromatische Polyamine sind z. B. geeignet : p-Phenylendiamin, 2,4-/2,6-Toluylendiamine, Diphenylmethan-4,4'- und/oder -2,4'- und/oder -2,2'-diamine, 3,3'-Dichlor-4,4'-diaminodiphenylmethan, 3-(C$_1$-C$_8$)-Alkyl-4,4'-diaminodiphenylmethane, die 3,3'-Di-(C$_1$-C$_4$)-4,4'-diaminodiphenylmethane sowie die 3,3',5,5'-Tetra-(C$_1$-C$_4$)-alkyl-4,4'-diaminodiphenylmethane, die 4,4'-Diaminodiphenyl-sulfide, -sulfoxide oder -sulfone, Ethergruppen aufweisende Diamine gemäß DE-OS 1 770 525 und 1 809 172 (US-PS 3 654 364 und 3 736 295), gegebenenfalls in 5-Stellung substituierte 2-Halogen-1,3-phenylendiamine (DE-OS 2 001 772, 2 025 896

EP 0 179 344 B1

und 2 065 869), Bisanthranilsäureester (DE-OS 2 040 644 und 2 160 590), 2,4-Diaminobenzoesäureester nach DE-OS 2 025 900, sowie durch eine oder zwei $(C_1-C_4)$-Alkylgruppen substituierte Toluylendiamine. Besonders bevorzugt sind 3,5-Diethyl-2,4- und/oder -2,6-diaminotoluol (besonders ihre technischen (80/20)- oder (65/35)-Isomerengemische), unsymmetrisch tetraalkylsubstituierte Diaminodiphenylmethane, z. B. 3,5-Diethyl-3'-5'-diisopropyl-4,4'-diaminodiphenylmethan und ihre Isomerengemische entsprechend DE-OS 2 902 090, 4,4'-Diaminobenzanilid, sowie 3,5-Diaminobenzoesäure-$(C_1-C_4)$-alkylester, 4,4'- und/oder 2,4-Diamino-diphenylmethan, sowie Naphthylen-1,5-diamin.

Es sind jedoch auch Diole oder Diamine mit zusätzlichen Gruppen einsetzbar, z. B. Adipinsäure-bis-(2-hydroxyethyl)-ester, Terephthalsäure-bis-(2-hydroxyethyl)-ester, Diolurethane, Diol-harnstoffe oder Polyole, welche Sulfonat- und/oder Phosphonatgruppen enthalten, z. B. 1,6-Hexamethylen-bis-(2-hydroxyethylurethan), 4,4'-Diphenylmethan-bis-(2-hydroxyethylharnstoff) oder das Addukt von Na-Bisulfit an Butendiol-1,4, bzw. dessen Alkoxylierungsprodukte. Weitere niedermolekulare Verbindungen werden ausführlich in der DE-OS 2 854 384 beschrieben.

Weitere, gegebenenfalls mitzuverwendende Kettenverlängerer oder Vernetzer sind beispielsweise Ethylendiamin, Propylendiamin, Hexan-1,6-diamin, 2,2,4-Trimethyl-1,6-diaminohexan, 2,5-Dimethyl-2,5-diaminohexan, 1,10-Decandiamin, 1,11-Undecandiamin, 1,12-Dodecandiamin, Bis-aminomethyl-hexahydro-4,7-methano-indan (TCD-Diamin), 1,3-Cyclohexandiamin, 1,4-Cyclohexandiamin, 1-Amino-3,3,5-trimethyl-5-aminomethyl-cyclohexan (Isophorondiamin), 2,4- und/oder 2,6-Hexahydrotoluylendiamin, 2,4'- und/oder 4,4'-Diaminodicyclohexylmethan, m- oder p-Xylylendiamin, Bis-(3-aminopropyl)-methylamin, Bis-N,N'-(3-aminopropyl)-piperazin und 1-Amino-2-amino-methyl-3,3,5-(3,5,5)-trimethylcyclopentan, 2,2-Dialkylpentan-1,5-diamine oder Triamine wie 1,5,11-Triaminoundecan, 4-Aminomethyl-1,8-diaminooctan, Lysinmethylester, cycloaliphatische Triamine gemäß DE-OS 2 614 244, 4,7-Dioxadecan-1,10-diamin, 2,4-und 2,6-Diamino-3,5-diethyl-1-methylcyclohexan und deren Gemische, alkylierte Diaminodicyclohexylmethane, z. B. 3,3'-Dimethyl-4,4'-diaminodicyclohexylmethan oder 3,5-Diisopropyl-3',5'-diethyl-4,4'-diaminodicyclohexylmethan, perhydrierte Diaminonaphthaline, perhydrierte Diaminoanthrazene, oder höherwertige Amine wie Diethylentriamin, Triethylentetramin, Pentaethylenhexamin, Dipropylentriamin, Tripropylentetramin, oder N,N'-Dimethyl-ethylendiamin, 2,5-Dimethylpiperazin, 2-Methylpiperazin, Piperazin-(hydrat) und 2-Hydroxyethylpiperazin.

Ferner sind als niedermolekulare Kettenverlängerungsmittel einsetzbar : Hydrazin, zumeist in Form von Hydrazinhydrat, $C_1-C_6$-alkylsubstituierte Hydrazine, z. B. Methylhydrazin, Ethylhydrazin, Hydroxyethyl-hydrazin oder N,N'-Dimethylhydrazin. Weiter geeignete Kettenverlängerer sind Verbindungen mit Hydrazid-Endgruppen, z. B. Di- oder Polyhydrazide wie Carbodihydrazid, Hydracrylsäurehydrazid, Oxalsäuredihydrazid, Adipinsäuredihydrazid, Terephthalsäuredihydrazid, Isophthalsäurehydrazid oder Verbindungen mit Hydrazid- und Semicarbazid-, Carbazinester- oder Amino-Gruppen, z. B. β-Semicarbazidopropionsäurehydrazid, Aminoessigsäurehydrazid und β-Aminopropionsäurehydrazid.

Ferner können in üblicher Weise gegebenenfalls gegenüber Isocyanaten monofunktionelle Verbindungen in Anteilen von 0,01 bis 10 Gew.-%, bezogen auf Polyurethanfeststoff, als sogenannte Kettenabbrecher mitverwendet werden. Derartige monofunktionelle Verbindungen sind z. B. Monoamine, wie Butyl- oder Dibutylamin, Stearylamin, Pyrrolidin, 4-Amino-2,2,6,6-tetramethylpiperidin, Anilin oder Tolylamin, Monoalkohole wie Butanol, 2-Ethylhexanol, Cyclohexanol, Ethylenglykolmonoethylester, Monooxime wie Butanonoxim oder andere monofunktionelle Verbindungen wie N,N'-Dimethylhydrazin oder Essigsäurehydrazid bzw. Benzoesäurehydrazid.

Als gegebenenfalls einzusetzende Katalysatoren C) für die erfindungsgemäß langzeitlagerstabilen Einkomponentensysteme können die üblichen Polyurethankatalysatoren, mit besonders gutem Effekt tertiäre Amine oder Metallkatalysatoren, verwendet werden.

Es sind dies z. B. tertiäre Amine, wie Triethylamin, Tributylamin, N-Methyl-morpholin, N-Ethylmorpholin, N-Cocomorpholin, N,N,N',N'-Tetramethyl-ethylendiamin, 1,4-Diaza-bicyclo-(2,2,2)-octan, N-Methyl-N'-dimethylaminoethyl-piperazin, N,N-Dimethylbenzylamin, Bis-(N,N-diethylaminoethyl)-adipat, N,N-Diethylbenzylamin, Pentamethyl-diethylentriamin, N,N-Dimethylcyclohexylamin, N,N,N',N'-Tetramethyl-1,3-butandiamin, N-N-Dimethyl-β-phenylethylamin, 1,2-Dimethylimidazol und 2-Methylimidazol.

Gegenüber Isocyanatgruppen aktive Wasserstoffatome aufweisende tertiäre Amine sind z. B. Triethanolamin, Triisopropanolamin, N-Methyl-diethanolamin, N-Ethyl-diethanolamin, Dimethyl-ethanolamin, sowie deren Umsetzungsprodukte mit Alkylenoxiden, wie Propylenoxid und/oder Ethylenoxid.

Als Katalysatoren kommen ferner Silaamine mit Kohlenstoff-Silizium-Bindungen, wie sie z. B. in der DE-PS 1 229 290 beschrieben sind, in Frage, z. B. 2,2,4-Trimethyl-2-silamorpholin und 1,3-Diethylaminomethyl-tetramethyl-disiloxan.

Als Katalysatoren kommen auch stickstoffhaltige Basen wie Tetraalkylammoniumhydroxide, ferner Alkalihydroxide wie Natriumhydroxid, Alkaliphenolate wie Natriumphenolat oder Alkalialkoholate wie Natriummethylat in Betracht. Auch Hexahydrotriazine können als Katalysator eingesetzt werden.

Es können auch organische Metallverbindungen, insbesondere organische Zinnverbindungen und Bleiverbindungen als Katalysatoren verwendet werden. Als organische Zinnverbindungen kommen vorzugsweise Zinn(II)-salze von Carbonsäuren wie Zinn(II)-acetat, Zinn(II)-octoat, Zinn(II)-ethylhexoat und Zinn(II)-laurat und die Dialkylzinnsalze von Carbonsäuren, wie z. B. Dibutyl-zinn-diacetat, Dibutylzinndilaurat, Dibutylzinn-maleat oder Dioctylzinndiacetat in Betracht.

Weitere Vertreter von erfindungsgemäß zu verwendenden Katalysatoren sowie Einzelheiten über die

10

Wirkungsweise der Katalysatoren sind im Kunststoff-Handbuch, Band VII, herausgegeben von Vieweg und Höchtlen, Carl-Hanser-Verlag, München 1966, z. B. auf den Seiten 96 bis 102, und in der EP-A 3 230 757 beschrieben.

Die Katalysatoren werden in der Regel in einer Menge zwischen etwa 0,001 und 10 Gew.-%, bezogen auf die Reaktivmischung eingesetzt.

Als gegebenenfalls einzusetzende Hilfs- und Zusatzstoffe D) seien genannt: Farbstoffe oder Pigmente, Füllstoffe wie Silicagel, Gips, Talkum, Aktivkohle, Metallpulver, UV-Absorptionsmittel oder Stabilisatoren wie phenolische Antioxidantien, Lichtschutzmittel, Treibmittel, oberflächenaktive Zusatzstoffe wie Emulgatoren oder Schaumstabilisatoren, gegebenenfalls Zellregler, Antiblockmittel, Silikone, Flammschutzmittel, oder fungistatisch und/oder bakteriostatisch wirkende Substanzen.

Als Füllstoffe sind z. B. Fasermaterialien, d. h. alle an sich bekannten anorganischen und/oder organischen, faserförmigen Verstärkungsmaterialien einsetzbar.

Sollen nach der erfindungsgemäßen Verwendung Polyurethanschaumstoffe hergestellt werden, dann werden Wasser und/oder leicht flüchtige organische Substanzen als Treibmittel mitverwendet. Als organische Treibmittel kommen z. B. Aceton, Ethylacetat, Methanol, Ethanol, halogensubstituierte Alkane wie Methylenchlorid, Chloroform, Ethylidenchlorid, Vinylidenchlorid, Monofluortrichlormethan, Chlordifluormethan, Dichlordifluormethan, ferner Butan, Hexan, Heptan oder Diethylether in Frage.

Eine Treibwirkung kann auch durch Zusatz von bei höheren Temperaturen unter Abspaltung von Gasen, beispielsweise von Stickstoff, sich zersetzenden Verbindungen, z. B. Azoverbindungen wie Azoisobuttersäurenitril, erzielt werden. Weitere Beispiele für Treibmittel sowie Einzelheiten über die Verwendung von Treibmitteln sind im Kunststoff-Handbuch, Band VII, herausgegeben von Vieweg und Höchtlen, Carl-Hanser-Verlag, München 1966, z. B. auf den Seiten 108 und 109, 453 und 455 und 507 bis 510 beschrieben.

Es können auch oberflächenaktive Zusatzstoffe (Emulgatoren und Schaumstabilisatoren) mitverwendet werden. Als Emulgatoren kommen z. B. die Natriumsalze von Ricinusölsulfonaten oder auch von Fettsäuren oder Salze von Fettsäuren mit Aminen wie ölsaures Diethylamin oder stearinsaures Diethanolamin in Frage. Auch Alkali- oder Ammoniumsalze von Sulfonsäuren wie etwa von Dodecylbenzolsulfonsäure oder Dinaphthylmethandisulfonsäure oder auch von Fettsäuren wie Ricinolsäure oder von polymeren Fettsäuren können als oberflächenaktive Zusatzstoffe mitverwendet werden.

Als Schaumstabilisatoren kommen vor allem wasserlösliche Polyethersiloxane in Frage. Diese Verbindungen sind im allgemeinen so aufgebaut, daß ein Copolymerisat aus Ethylenoxid und Propylenoxid mit einem Polydimethylsiloxanrest verbunden ist. Derartige Schaumstabilisatoren sind z. B. in der US-PS 2 764 565 beschrieben.

Es können ferner auch Reaktionsverzögerer, z. B. sauer reagierende Stoffe wie Salzsäure oder organische Säurehalogenide, ferner Zellregler der an sich bekannten Art wie Paraffine oder Fettalkohole oder Dimethylpolysiloxane sowie Pigmente oder Farbstoffe und Flammschutzmittel der an sich bekannten Art, z. B. Tris-chlorethylphosphat oder Ammoniumphosphat und -Polyphosphat, ferner Stabilisatoren gegen Alterungs- und Witterungseinflüsse, Weichmacher und/oder fungistatisch und bakteriostatisch wirkende Substanzen, Füllstoffe wie Bariumsulfat, Kieselgur, Ruß oder Schlämmkreide mitverwendet werden.

Weitere Beispiele von gegebenenfalls erfindungsgemäß mitzuverwendenden oberflächenaktiven Zusatzstoffen und Schaumstabilisatoren sowie Zellreglern, Reaktionsverzögerern, Stabilisatoren, flammhemmenden Substanzen, Weichmachern, Farbstoffen und Füllstoffen sowie fungistatisch und bakteriostatisch wirksamen Substanzen sowie Einzelheiten über Verwendungs- und Wirkungsweise dieser Zusatzmittel sind im Kunststoff-Handbuch, Band VI, herausgegeben von Vieweg und Höchtlen, Carl-Hanser-Verlag, München 1966, z. B. auf den Seiten 103 bis 113 und in den DE-OSen 2 854 384 und 2 920 501 beschrieben.

Die Reaktionskomponenten werden z. B. nach dem an sich bekannten Einstufenverfahren, dem Prepolymerverfahren oder dem Semiprepolymerverfahren, in der Regel bei Kennzahlen von 50 bis 300, vorzugsweise 95 bis 250, insbesondere 100 bis 130 zur Umsetzung gebracht, wobei man sich oft maschineller Einrichtungen bedient, z. B. solcher, die in der US-PS 2 764 565 beschrieben werden. Einzelheiten über Verarbeitungseinrichtungen, die auch erfindungsgemäß in Frage kommen, werden im Kunststoff-Handbuch, Band VII, herausgegeben von Vieweg und Höchtlen, Carl-Hanser-Verlag, München 1966, z. B. auf den Seiten 121 bis 205 beschrieben.

Bei der Schaumstoffherstellung kann erfindungsgemäß die Verschäumung auch in geschlossenen Formen durchgeführt werden. Dabei wird das Reaktionsgemisch in eine Form eingetragen. Als Formmaterial kommt Metall, z. B. Aluminium, oder Kunststoff, z. B. Epoxidharz, in Frage. In der Form schäumt das schaumfähige Reaktionsgemisch auf und bildet den Formkörper. Die Formverschäumung kann dabei so durchgeführt werden, daß das Formteil an seiner Oberfläche Zellstruktur aufweist, sie kann aber auch so durchgeführt werden, daß das Formteil eine kompakte Haut und einen zelligen Kern aufweist. Erfindungsgemäß kann man in diesem Zusammenhang so vorgehen, daß man in die Form so viel schäumfähiges Reaktionsgemisch einträgt, daß der gebildete Schaumstoff die Form gerade ausfüllt. Man kann aber auch so arbeiten, daß man mehr schäumfähiges Reaktionsgemisch in die Form einträgt, als zur Ausfüllung des Forminneren mit Schaumstoff notwendig ist. Im letztgenannten Fall wird somit unter « overcharging » gearbeitet.

Bei der Formverschäumung werden vielfach an sich bekannte « äußere Trennmittel », wie Siliconöle, oder sogenannte « innere Trennmittel », gegebenenfalls im Gemisch mit äußeren Trennmitteln, verwendet.

Selbstverständlich können aber auch Schaumstoffe durch Blockverschäumung oder nach dem an sich bekannten Doppeltransportbandverfahren hergestellt werden.

Die nach der Erfindung erhältlichen Produkte finden z. B. als Dämmplatten für die Dachisolierung oder als Automobilteile Anwendung.

Herstellung von Katalysatoren der Gruppe a)

Katalysator A

Entsprechend der DE-AS 1 039 232 werden 5 856 Gewichtsteile Thiodiglykol mit 29,3 Gewichtsteilen ortho-Phosphorsäure versetzt. Es wird so lange kondensiert, bis 765 ml Wasser abgespalten sind. Anschließend legt man 3 Stunden bei 180 °C Wasserstrahlvakuum an und erhält in 91 % Ausbeute ein Reaktionsprodukt mit einer OH-Zahl 110. Der erhaltene Polythioether hat ein Molekulargewicht von etwa 1 000.

Katalysator B

Zur Herstellung eines Polythioethers mit einem Molekulargewicht von 2 000 wird entsprechend der Herstellung des Katalysators A gearbeitet. Es muß jedoch 3 Stunden länger Wasser (insgesamt 820 ml) abgeschieden werden. Man erhält ein Produkt mit einem Molekulargewicht von ca. 2 000 und einer OH-Zahl von 54.

Herstellung der Katalysatoren der Gruppe b) am Beispiel des Katalysators C

In einem 2 l-Rundkolben werden 780 g 2-Mercaptoethanol und 545 g Limonen unter gleichzeitigem Einleiten von Luft 24 Stunden bei Raumtemperatur gerührt. Danach destilliert man überschüssiges Mercaptoethanol im Vakuum ab. Man erhält ein Diaddukt von Mercaptoethanol an Limonen mit folgenden analytischen Daten

OH-Zahl 381
Viskosität : bei 25 °C/1 300 mPas

Kondensation des Katalysators C zu einem Polythioether mit einem Molgewicht von ca. 1 100

4 Mol des Katalysators C werden mit 5,8 g konz. $H_3PO_4$ versetzt und 6 Stunden im Wasserstrahlvakuum auf 160 °C erhitzt. Hierbei destillierten 52 g Wasser ab. Das so erhaltene Produkt wies eine OH-Zahl von 101 auf. Die Viskosität betrug 7 500 mPas/25 °C.

Beispiel 1 (Stufe A)

34,4 kg (400 Mol) Butindiol-1,4 werden in einem 100 l Kessel aufgeschmolzen (Fp. ca. 58 °C) und nach Zugabe von 242 g Kaliumchlorid wird bei 70 °C und 2 bar Stickstoffdruck innerhalb von 6 bis 7 Stunden 46,4 kg (800 Mol) Propylenoxid zudosiert. Es wird dann 5 Stunden bei 80° nachreagiert. Nach Zugabe von 80 g 2,6-Di-t-butyl-4-methylphenol wird zur Entfernung geringer Mengen freien Propylenoxids 1 Stunde bei 80 °C im Vakuum entgast und dann von ausgefallenem Kaliumchlorid abfiltriert.

Ausbeute : 80 kg
Die analytischen Daten sind wie folgt :

| | |
|---|---|
| OH-Zahl | 549 |
| $\eta_{25°}$ (mPas) | 149 |
| Säurezahl | 0,1 |
| $H_2O$ (%) | 0,05 |
| freies Butindiol (%) | 0,3 |

(Weiterverarbeitung siehe Beispiel 7).

Beispiel 2 (Stufe A)

104,2 kg (400 Mol) 33 %iges wäßriges Butindiol wird in einem 100 l Kessel bei max. 80 °C im Vakuum bis auf einen Wassergehalt von ≤ 0,5 % entwässert. Nach Zugabe von 210 g eines Polythioethers (Katalysator A) vom Molgewicht 1 000 werden bei 70 °C und 2 bar Stickstoffdruck innerhalb von 5 bis 6 Stunden 36,08 kg (820 Mol) Ethylenoxid zudosiert. Nach 5 Stunden Nachreaktion bei 80 °C und Zugabe von 70 g 2,6-Di-t-butyl-4-methylphenol wird zur Entfernung geringer Mengen unreagierten Ethylenoxids 1 Stunde im Vakuum entgast. Ausbeute : 70 kg.

EP 0 179 344 B1

Die analytischen Daten sind wie folgt :

| | |
|---|---|
| OH-Zahl | 619 |
| $\eta_{25°}$ (mPas) | 90 |
| Säurezahl | 0,03 |
| $H_2O$ (%) | 0,05 |
| freies Butindiol (%) | 0,5 |

(Weiterverarbeitung siehe Beispiel 8)

### Beispiel 3 (Stufe A)

104,2 kg (400 Mol) 33 %iges wäßriges Butindiol wird in einem 100 l Kessel bei max. 80 °C im Vakuum bis auf einen Wassergehalt von ≤ 0,5 % entwässert. Nach Zugabe von 280 g eines Polythioethers vom Molgewicht 2 000 (Katalysator B) wird bei 70 °C und 2 bar Stickstoffdruck ein Gemisch von 4,4 kg (100 Mol) Ethylenoxid und 5,8 kg (100 Mol) Propylenoxid innerhalb von 2 Stunden zudosiert. Nach 2 Stunden Nachreaktion folgen innerhalb von 5 bis 6 Stunden 26,4 kg (600 Mol) Ethylenoxid. Nach 5 Stunden Nachreaktion bei 80 °C und Zugabe von 70 g 2,6-Di-t-butyl-4-methylphenol wird zur Entfernung geringer Mengen Ethylenoxids 1 Stunde im Vakuum entgast. Ausbeuten : 70,5 kg. Die analytischen Daten sind wie folgt :

| | |
|---|---|
| OH-Zahl | 622 |
| $\eta_{25°}$ (mPas) | 105 |
| Säurezahl | 0,1 |
| $H_2O$ (%) | 0,04 |
| freies Butindiol (%) | 0,6 |

(Weiterverarbeitung siehe Beispiel 9).

### Beispiel 4 (Stufe A, Vergleichsbeispiel)

In einem 2 l Rundkolben mit Rührer, Kühler, Tropftrichter und Thermometer werden 344 g (4 Mol) Butindiol in 320 g Aceton gelöst. Nach Zugabe von 2 ml $BF_3$-Etherat läßt man bei 20 °C 348 g (6 Mol) Propylenoxid langsam zutropfen. Nach Abdestillieren des Acetons erhält man ein Addukt mit der OH-Zahl 651, welches noch 23 Gew.-% freies Butindiol enthält.

Isomerenverteilung

| | |
|---|---|
| freies Butindiol | 23 % |
| Butindiol + 1 PO | 34 % |
| Butindiol + 2 PO | 26 % |
| Butindiol + 3 PO | 16 % |

(Weiterverarbeitung siehe Beispiel 10).

### Beispiel 5

In einem weiteren Versuch werden 344 g (4 Mol) Butindiol und 3,5 g eines Polythioethers vom Molgewicht 1 000 (Katalysator A) bei 70° mit 348 g (6 Mol) Propylenoxid allmählich versetzt. Nach einer Nachreaktion bei 80 °C erhält man ein Addukt der OH-Zahl 649, welches 4,4 Gew.-% freies Butindiol enthält.

Isomerenverteilung

| | |
|---|---|
| freies Butindiol | 4,4 % |
| Butindiol + 1 PO | 37,9 % |
| Butindiol + 2 PO | 56,2 % |
| Butindiol + 3 PO | 1,4 % |

### Beispiel 6 (Stufe A)

860 g (10 Mol) Butindiol-1,4 werden in einem 5 l-Kolben aufgeschmolzen und nach Zugabe von 8,8 g 1-Methyl-2-(2-Hydroxyethylthio)-4-(1-methyl-2-(2-Hydroxyethylthio)-ethylcyclohexan (Katalysator C) werden bei 70° und 0,5 bar Stickstoff 902 g (20,5 Mol) Ethylenoxid innerhalb von 6 Stunden zudosiert. Nach 5 Stunden Nachreaktion und Zugabe von 2 g 2,6-Di-t-butyl-4-methylphenol wird zur Entfernung geringer Mengen freien Ethylenoxids 1 Stunde bei 70° im Vakuum entgast.

Ausbeute : 1 760 g

Die analytischen Daten sind wie folgt :

| | |
|---|---|
| OH-Zahl | 636 |
| $\eta_{25°}$ (mPas) | 88 |
| Säurezahl | 0,07 |
| $H_2O$ (%) | 0,06 |
| freies Butindiol (%) | < 0,2 % |

## Beispiel 7 (Stufen B-E)

In einem 100 l Email-Kessel werden 51,93 kg eines Polyethers aus Beispiel 1 (OH-Zahl : 549 ; F = 2) bei 10° unter Stickstoff vorgelegt. Nun dosiert man 40,6 kg Brom, so zu, daß die Temperatur durch Kühlung nicht über 20 °C steigt. Nach ca. 6 h ist die Bromdosierung beendet und man rührt noch 2 h bei 20 °C nach. Entsprechend dem Gehalt an entstandenem Bromwasserstoff wird anschließend bei angelegter Kühlung noch 3,2 kg einer 34 %igen Wasserstoffperoxidlösung (110 % der theoretisch benötigten Menge) zugetropft. (Temp. ≤ 20 °C). Zur Nachreaktion läßt man 12 h bei Raumtemperatur rühren, versetzt dann mit 1 kg Epoxid Levepox 4020(R) (BAYER AG, D-5090 Leverkusen) und entwässert im Vakuum bei 70 °C. Der Polyether bleibt homogen und stabil und ist geruchsfrei.

Ausbeute : 90,7 kg
Analytische Daten :

| | |
|---|---|
| OH-Zahl | 296 |
| Säurezahl | 0,2 |
| pH | 6,8 |
| $H_2O$ | 0,05 % |
| $\eta_{25°}$ | 580 mPas |

## Beispiel 8 (Stufen B-E)

Entsprechend Beispiel 7 werden in einem 100 l Kessel 59,00 kg des Polyethers aus Beispiel 2 (325,5 Mol) und 52,08 kg Brom zur Reaktion gebracht. Der entstandene HBr wird mit 2,46 kg 34 %iger Wasserstoffperoxidlösung aufoxidiert und anschließend bei 70 °C im Vakuum entwässert. Zur Stabilisierung wird 1,1 kg Epoxid Levepox 4020(R) eingesetzt. Der Polyether bleibt homogen und stabil und ist praktisch geruchsfrei.

Ausbeute : 109 kg
Analytische Daten :

| | |
|---|---|
| Säurezahl | 0,2 |
| OH-Zahl | 325 |
| $H_2O$ | 0,05 % |
| pH | 6,9 |
| $\eta_{25°}$ | 410 mPas |

## Beispiel 9 (Stufen B-E)

Entsprechend Beispiel 7 werden 50,51 kg des Polyethers aus Beispiel 3 mit 45,67 kg Brom zur Reaktion gebracht. Der entstandene Bromwasserstoff wird mit 2,67 kg 34 %iger Wasserstoffperoxidlösung (10 %iger Überschuß) aufoxidiert und im Wasserstrahlvakuum bei 70 °C entwässert. Der Ansatz wird mit 1 kg Levepox 4020(R) stabilisiert. Der Polyether bleibt homogen und stabil und ist praktisch geruchsfrei.

Ausbeute : 94,5 kg
Analytische Daten :

| | |
|---|---|
| Säurezahl | 0,3 |
| OH-Zahl | 320 |
| $H_2O$ | 0,1 % |
| pH | 6,5 |
| $\eta_{25°}$ | 470 mPas |

## Beispiel 10 (Stufen B-E/Vergleichsbeispiel)

606 g des Polyethers aus Beispiel 4 mit einem hohen Gehalt an Butindiol werden mit 560 g Brom bei einer Temperatur von 15 bis 20 °C umgesetzt. Das entstehende Produkt wird entsprechend den Beispielen 7-9 mit Wasserstoffperoxidlösung behandelt, entwässert und mit Levepox 4020(R) stabilisiert. Das durch die Umsetzung mit Brom entstandene Dibrombutendiol kristallierte nach der Reaktion aus. Der so erhaltene Polyether hatte nach einem Tag eine breiige Konsistenz und war nicht mehr fließfähig.

## Beispiel 11

2 279 g des Polyethers aus Beispiel 5 werden mit 2 560 g Brom bei 20 °C umgesetzt. Das Rohprodukt wird entsprechend den vorherigen Beispielen aufgearbeitet. Man erhält 5 007 g klares Produkt, das auch bei Lagerung homogen und stabil bleibt, mit folgenden analytischen Daten :

| | |
|---|---|
| Säurezahl | 0,3 |
| $H_2O$ | 0,12 % |
| OH-Zahl | 307 |
| $\eta_{25°}$ | 710 mPas |

## Beispiel 12

1 058 g (6 Mol) des Polyethers aus Beispiel 6 werden entsprechend Beispiel 7 mit 960 g Brom umgesetzt. Man erhält in fast quantitativer Ausbeute einen Polyether mit folgenden analytischen Daten :

| | |
|---|---|
| Säurezahl | 0,1 |
| OH-Zahl | 323 |
| $H_2O$ | 0,2 % |
| $\eta_{25°}$ | 450 mPas |

## Beispiel 13 (Vergleichsbeispiel, Alkoxylierung nach Beispiel 1 der DE-OS 2 241 156)

860 g (10 Mol) Butindiol-1,4 werden entsprechend Beispiel 6 mit 902 g (20,5 Mol) Ethylenoxid unter Katalyse von 8,6 g Thiodiglykol bei 55-58 °C umgesetzt. Man erhielt 1 750 g des ethoxylierten Produktes.
Die analytischen Daten waren wie folgt :

| | |
|---|---|
| OH-Zahl | 634 |
| $\eta_{25°}$ | 88 mPas |
| Säurezahl | 0,07 |
| $H_2O$ | 0,1 |
| freies Butindiol-1,4 | 2,5 % |

Das so erhaltene Alkoxylierungsprodukt wurde mit 10 Mol Brom entsprechend Beispiel 7 umgesetzt. Das erhaltene Produkt und insbesondere die bei der Destillation anfallenden Abwässer wiesen einen unangenehmen Geruch auf. Bei Zugabe von 20 % Frigen fiel das freie Dibrombutendiol aus.

### Anwendungsbeispiele der erfindungsgemäß hergestellten, bromierten Polyether

Für die Herstellung der erfindungsgemäßen Schaumstoffe wurden folgende Dibrombutendiolpolyether eingesetzt :

Ether 1 (Beispiel 8) : 1 Mol Dibrombutendiol + 2,05 Mol Ethylenoxid, OH-Zahl 325
Ether 2 (Beispiel 9) : 1 Mol Dibrombutendiol + 1,75 Mol Ethylenoxid + 0,25 Mol Propylenoxid, OH-Zahl 320

(Siehe Tabelle 1 Seite 16 f.)

Tabelle 1

| Beispiel | 14 | 15 | 16 | 17 | 18 |
|---|---|---|---|---|---|
| Dimethylcyclohexylamin [1] | 0,3 | 0,5 | 1 | 2 | 1,8 |
| 25 % Kaliumacetat in Diethylenglykol [1] | 2 | 2 | – | – | – |
| Propylenoxidpolyether, Startermaterial Zucker und Wasser, OH-Zahl 465, Funktionalität 4,3 [1] | 40 | 55 | 45 | 50 | 60 |
| Dimethylmethanphosphonat [1] | – | – | – | 5 | 7 |
| Trichlorethylphosphat [1] | – | 15 | – | – | – |
| Diethyl-N,N-bis-(2-hydroxy-ethyl)-aminomethylphosphonat [1] | – | – | – | 15 | – |
| Ether 1 [1] | 60 | 30 | – | – | – |
| Ether 2 [1] | – | – | 55 | 30 | 33 |
| Siliconstabilisator B 8404 von Fa. Goldschmidt / Essen [1] | 1 | 1 | 1 | | 1 |
| Wasser [1] | – | – | 0,5 | – | 0,5 |
| Trichlorfluormethan [1] | 40 | 40 | 35 | 35 | 35 |
| Diese Komponenten werden vorvermischt | | | | | |
| 4,4'-Diphenylmethandiisocyanat [1] mit polymeren Anteilen (Desmodur(R) 44-V20, BAYER AG, D-5090 Leverkusen) | 200 | 200 | 111 | 114 | 110 |
| Abbindezeit (sec.) | 60 | 60 | 60 | 60 | 60 |
| Kennzahl | 210 | 226 | 110 | 110 | 110 |
| Rohdichte (kg/m$^3$) | 37 | 37 | 26 | 25 | 25 |
| Schrumpf | kein | kaum | kaum | kaum | kein |
| Flammenhöhe (mm) im Schweizer BVD-Test [2] | 120 | 128 | 167 | 120 | 167 |
| Brandklasse im BVD-Test [2] | V | V | II | V | II |
| Brandklasse nach DIN 4102 | B2 | B2 | B2 | B2 | B2 |

[1] Gew.-Teile
[2] BVD : « Brand-Verhütungs-Dienst »
aus
« Bestimmung der Kennziffer gemäß Wegleitung für Feuerpolizeivorschriften, Prüfung von Brennstoffen und Bauelementen (Brennbarkeit und Qualmbildung) »
Ausgabe 1976

Die Schaumstoffe wurden auf folgende Weise hergestellt: Alle Komponenten, Isocyanat ausgenommen, werden vermischt und anschließend 10 sec intensiv mit dem Isocyanat gerührt. Die Bezeichnung « kein » oder « kaum » unter der Rubrik Schrumpf ist ein Zeichen für die Durchhärtung der Schaumstoffe, die Bezeichnung « kein » bedeutet also eine bessere Durchhärtung.

Beim Brandverhalten wird gezeigt, daß durch Kombination mit Phosphor-enthaltenden Flammschutzmitteln ein besseres Ergebnis beim Brandtest (höhere Flammwidrigkeit) oder aber bei gleichem Ergebnis im Brandtest eine Reduzierung der notwendigen Zusatzmenge an Flammschutzmitteln erreicht wird.

**Patentansprüche**

1. Verfahren zur Herstellung von lagerstabilen, flüssigen, bromhaltigen, 2,3-Dibrombutendiol-(1,4)-armen Alkoxylierungsprodukten aus Butindiol-1,4 unter katalytischer Anlagerung von Epoxiden und anschließender Bromierung, dadurch gekennzeichnet, daß man

A) 2,3-Butindiol-(1,4) mit 1,5 bis 2,5 Mol an Alkylenoxiden, in Gegenwart eines Katalysators aus der Gruppe

a) der höhermolekularen Thioether mit einem Molekulargewicht von 500 bis 5 000, wie sie durch Kondensation von Thiodiglykol, vorzugsweise durch saure Kondensation, erhalten werden und/oder

b) von Thioethern, wie sie durch Anlagerung von Mercaptanen und/oder Mercaptoalkoholen an ungesättigte Kohlenwasserstoffe erhalten werden, oder ihren Kondensationsprodukten zu höhermolekularen Thioethern mit Molekulargewichten bis 5 000, oder

c) eines Alkalichlorids,

bei erhöhten Temperaturen umsetzt,

B) das entsprechende Butindiol-(1,4)-arme Alkoxylierungsprodukt bei —10 °C bis 80 °C, vorzugsweise bei 0 °C bis 40 °C, besonders bevorzugt bei 0 °C bis 20 °C, mit 0,6 bis 1,9 Mol, vorzugsweise 0,98 bis 1,02 Mol, Brom pro Mol Butindiol umsetzt.

C) den in einer Nebenreaktion entsprechenden Bromwasserstoff durch Rückoxidation mit wäßriger Wasserstoffperoxidlösung in reaktives Brom überführt,

D) mit einer schwerflüchtigen Epoxidverbindung, vorzugsweise in Mengen von 0,3 bis 3 Gew.-%, stabilisiert und

E) das bromierte Endprodukt nach Stufe C) oder D) entwässert.

2. Verfahren nach Anspruch 1, dadurch gekennzeichnet, daß man als höhermolekulare Thioether a) durch saure Kondensation von Thiodiglykol mittels konzentrierter Phosphorsäure erhaltene Thioether einsetzt.

3. Verfahren nach Anspruch 1, dadurch gekennzeichnet, daß man als Thioether b) Additionsprodukte von Mercaptoethanol an Limonen einsetzt.

4. Verfahren nach Ansprüchen 1-3, dadurch gekennzeichnet, daß man Butindiol-1,4 mit 1,98-2,05 Mol an Ethylenoxid und/oder Propylenoxid in Stufe A umsetzt.

5. Verfahren nach Ansprüchen 1-4, dadurch gekennzeichnet, daß man den in der Bromierungsstufe B) anfallenden Bromwasserstoff mit etwa äquivalenten oder leicht überschüssigen Mengen Wasserstoffperoxid, vorzugsweise bei Temperaturen von 0-40 °C, rückoxidiert.

6. Verfahren nach Ansprüchen 1-5, dadurch gekennzeichnet, daß man das Verfahrensprodukt durch Zusatz von schwerflüchtigen Epoxiden mit Molekulargewichten oberhalb 174 stabilisiert.

7. Verwendung der bromhaltigen Alkoxylierungsprodukte nach Ansprüchen 1-6 in einem Verfahren zur Herstellung von flammwidrigen Kunststoffen auf Polyisocyanatbasis, vorzugsweise flammwidrigen PU-Schaumstoffen einschließlich Polyisocyanurat-Schaumstoffen, durch Umsetzung von organischen Polyisocyanaten mit höhermolekularen Verbindungen mit gegenüber NCO reaktiven Gruppen mit reaktiven H-Atomen, z. B. OH—, NH₂—, NHR—, COOH und/oder SH-Gruppen, vorzugsweise höhermolekularen Polyhydroxylverbindungen, gegebenenfalls Wasser und/oder niedermolekularen Kettenverlängerungsmitteln und/oder Vernetzern in Gegenwart der in der PU-Chemie üblichen Hilfs- und Zusatzstoffe, Katalysatoren einschließlich, Trimerisierungskatalysatoren, dadurch gekennzeichnet, daß man als höhermolekulare Polyhydroxylverbindungen, zumindest teilweise in Mengen von 1-60 Gewichtssprozent, die bromhaltigen Alkoxylierungsprodukte, gegebenenfalls unter Zumischung anderer einbaufähiger oder nicht einbaufähiger Flammschutzmittel einsetzt.

8. Verwendung nach Anspruch 7, dadurch gekennzeichnet, daß man als Polyhydroxylverbindung 3 bis 50 Gew.-% der bromhaltigen Alkoxylierungsprodukte nach Ansprüchen 1-6 (bezogen auf höhermolekulare Polyolkomponente) einbringt, und, gegebenenfalls in Gegenwart von Wasser, in einem NCO-Kennzahlbereich von 100 bis 135 flammwidrige Polyurethan (harnstoff)-Schaumstoffe herstellt.

9. Verwendung nach Anspruch 7, dadurch gekennzeichnet, daß man als Polyhydroxylverbindung 3 bis 50 Gew.-% der bromhaltigen Alkoxylierungsprodukte nach Ansprüchen 1-6 einbringt und in einem NCO-Kennzahlbereich von 135 bis 500 in Gegenwart von Trimerisierungskatalysatoren flammwidrige Polyisocyanurat-Schaumstoffe herstellt.

10. Verwendung nach Ansprüchen 7-9, dadurch gekennzeichnet, daß man neben den erfindungsgemäß einzusetzenden bromhaltigen Alkoxylierungsprodukten zusätzlich 3-25 Gew.-% phosphorhaltiger und/oder halogen-, insbesondere brom-haltiger, einbaufähiger oder nicht einbaufähiger Flammschutzmittel mitverwendet.

## Claims

1. A process for the production of storable, liquid, bromine-containing alkoxylation products of low 2,3-di-bromobutene-1,4-diol content from butyne-1,4-diol with catalytic addition of epoxides and subsequent bromination, characterized in that

A) 2,3-butyne-1,4-diol is reacted with 1.5 to 2.5 mol of alkylene oxides in the presence of a catalyst from the group consisting of

a) relatively high molecular weight thioethers having a molecular weight of from 500 to 5 000 of the type obtained by condensation of thiodiglycol, preferably by acidic condensation, and/or

b) thioethers of the type obtained by addition of mercaptans and/or mercaptoalcohols onto unsaturated hydrocarbons or condensates thereof to relatively high molecular weight thioethers having molecular weights up to 5 000, or

c) an alkali chloride, at elevated temperatures,

B) the corresponding alkoxylation product of low butyne-1,4-diol content is reacted at — 10 to 80 °C, preferably at 0 to 40 °C and more preferably at 0 to 20 °C with from 0.6 to 0.9 mol and preferably with from 0.98 to 1.02 mol bromine per mol butyne diol,

C) the hydrogen bromide formed in a secondary reaction is converted into reactive bromine by re-oxidation with aqueous hydrogen peroxide solution,

D) stabilized with a low-volatility epoxide compound in quantities of from 0.3 to 3 % by weight and

E) the brominated end product is dehydrated after step C) or D).

2. A process as claimed in claim 1, characterized in that thioethers obtained by acidic condensation of thiodiglycol with concentrated phosphoric acid are used as the relatively high molecular weight thioethers a).

3. A process as claimed in claim 1, characterized in that addition products of mercaptoethanol with limonene are used as the thioethers b).

4. A process as claimed in claims 1 to 3, characterized in that butyne-1,4-diol is reacted with 1.98 to 2.05 mol ethylene oxide and/or propylene oxide in step A.

5. A process as claimed in claims 1 to 4, characterized in that the hydrogen bromide formed in the bromination step B) is re-oxidized with substantially equivalent or slightly excess quantities of hydrogen peroxide, preferably at temperatures in the range from 0 to 40 °C.

6. A process as claimed in claims 1 to 5, characterized in that the end product of the process is stabilized by addition of low-volatility epoxides having molecular weights above 174.

7. The use of the bromine-containing alkoxylation products obtained in accordance with claims 1 to 6 in a process for the production of flame-resistant polyisocyanate-based plastics, preferably flame-resistant PU foams, including polyisocyanurate foams, by reaction of organic polyisocyanates with relatively high molecular weight compounds containing NCO-reactive groups containing reactive hydrogen atoms, for example OH—, NH$_2$—, NHR—, COOH— and/or SH-groups, preferably relatively high molecular weight polyhydroxyl compounds, optionally water and/or relatively low molecular weight chain-extending agents and/or crosslinking agents in the presence of the auxiliaries and additives and catalysts, including trimerization catalysts, typically used in PU chemistry, characterized in that the bromine-containing alkoxylation products are used at least partly in quantities of 1 to 60 % by weight as the relatively high molecular weight polyhydroxyl compounds, optionally with inclusion of other incorporable or non-incorporable flameproofing compounds.

8. The use claimed in claim 7, characterized in that 3 to 50 % by weight of the bromine-containing alkoxylation products according to claims 1 to 6 (based on relatively high molecular weight polyol component) are introduced as the polyhydroxyl compound and flame-resistant polyurethane (urea) foams are produced, optionally in the presence of water, in an NCO index range of 100 to 135.

9. The use claimed in claim 7, characterized in that 3 to 50 % by weight of the bromine-containing alkoxylation products claimed in claims 1 to 6 are introduced as the polyhydroxyl compound and flame-resistant polyisocyanurate foams are produced in the presence of trimerization catalysts in an NCO index range of 135 to 500.

10. The use claimed in claims 7 to 9, characterized in that 3 to 25 % by weight phosphorus-containing and/or halogen-containing, more especially bromine-containing, incorporable or non-incorporable flameproofing agents are used in addition to the bromine-containing alkoxylation products used in accordance with the invention.

**Revendications**

1. Procédé de préparation de produits d'alcoxylation stables à la conservation, liquides, contenant du brome, à faible teneur en 2,3-dibromo-butène-diol-1,4, du butyne-diol-1,4, par fixation catalytique par addition d'époxydes suivie d'une bromuration, caractérisé en ce que

A) on fait réagir à température élevée le 2,3-butyne-diol-1,4 avec 1,5 à 2,5 moles d'oxydes d'alkylène en présence d'un catalyseur du groupe consistant en

a) les thioéthers à haut poids moléculaire, ayant un poids moléculaire de 500 à 5 000, tels qu'obtenus par condensation du thiodiglycol, de préférence par condensation acide, et/ou

b) les thioéthers tels qu'obtenus par fixation par addition de mercaptans et/ou de mercapto-alcools sur des hydrocarbures insaturés, ou leurs produits de condensation en thioéthers à haut poids moléculaire, à des poids moléculaires allant jusqu'à 5 000, ou bien

c) un chlorure alcalin,

B) on fait réagir le produit d'alcoxylation obtenu, à faible teneur en butyne-diol-1,4, à des températures de — 10 à + 80 °C, de préférence de 0 à 40 °C et plus spécialement de 0 à 20 °C, avec 0,6 à 1,9 mole, de préférence 0,98 à 1,02 mole de brome par mole de butyne-diol.

C) on convertit le bromure d'hydrogène formé dans une réaction secondaire par oxydation en retour à l'aide d'une solution aqueuse de peroxyde d'hydrogène, en brome réactif.

D) on stabilise à l'aide d'un époxyde à faible volatilité, de préférence en quantité de 0,3 à 3 % en poids et

E) on déshydrate le produit final bromé après le stade opératoire C) ou le stade opératoire D).

2. Procédé selon la revendication 1, caractérisé en ce que l'on utilise en tant que thioéthers à haut poids moléculaire a) des thioéthers obtenus par condensation acide du thiodiglycol sous l'action de l'acide phosphorique concentré.

3. Procédé selon la revendication 1, caractérisé en ce que l'on utilise en tant que thioéthers b) des produits d'addition du mercaptoéthanol sur le limonène.

4. Procédé selon les revendications 1 à 3, caractérisé en ce que l'on fait réagir au stade opératoire A) le butyne-diol-1,4 avec 1,98 à 2,05 moles d'oxyde d'éthylène et/ou d'oxyde de propylène.

5. Procédé selon les revendications 1 à 4, caractérisé en ce que l'on oxyde en retour le bromure d'hydrogène formé au stade bromuration B) par une quantité à peu près équivalente ou un léger excès de peroxyde d'hydrogène, de préférence à des températures de 0 à 40 °C.

6. Procédé selon les revendications 1 à 5, caractérisé en ce que l'on stabilise le produit formé par addition d'époxydes à faible volatilité ayant des poids moléculaires supérieurs à 174.

7. Utilisation des produits d'alcoxylation bromés selon les revendications 1 à 6 dans un procédé de préparation de résines synthétiques ignifuges à base de polyisocyanates, de préférence des mousses de polyuréthannes ignifuges, y compris les mousses de polyisocyanurates, par réaction de polyisocyanates organiques avec des composés à haut poids moléculaire portant des groupes réactifs à atomes d'hydrogène réactifs à l'égard des groupes NCO, par exemple des groupes OH, $NH_2$, NHR, COOH et/ou SH, de préférence des composés polyhydroxylés à haut poids moléculaire, le cas échéant de l'eau et/ou des agents d'allongement des chaînes à bas poids moléculaire et/ou des agents réticulants en présence des produits auxiliaires et additifs usuels dans la chimie des polyuréthannes, de catalyseurs, y compris de catalyseurs de trimérisation, caractérisée en ce que l'on utilise en tant que composés polyhydroxylés à haut poids moléculaire, en partie au moins, en quantités de 1 à 60 % en poids, les produits d'alcoxylation bromés, éventuellement en mélange avec d'autres agents ignifugeants chimiquement combinables ou non chimiquement combinables.

8. Utilisation selon la revendication 7, caractérisée en ce que l'on utilise en tant que composés polyhydroxylés, en proportions de 3 à 50 % en poids, les produits d'alcoxylation bromés des revendications 1 à 6 (par rapport au composant polyol à haut poids moléculaire) et on prépare, le cas échéant en présence d'eau, et dans un domaine de 100 à 135 pour la caractéristique de NCO, des mousses de polyuréthanne-(urée) ignifuges.

9. Utilisation selon la revendication 7, caractérisée en ce que l'on utilise en tant que composés polyhydroxylés, en proportions de 3 à 50 % en poids, les produits d'alcoxylation bromés des revendications 1 à 6 et on prépare, dans un domaine de 135 à 500 pour la caractéristique de NCO, en présence de catalyseurs de trimérisation, des mousses de polyisocyanurates ignifuges.

10. Utilisation selon les revendications 7 à 9, caractérisée en ce que, avec les produits d'alcoxylation bromés selon l'invention, on utilise en outre de 3 à 25 % en poids d'agents ignifugeants phosphorés et/ou halogénés, en particulier bromés, combinables chimiquement ou non combinables chimiquement.